# EUROPÄISCHE PATENTSCHRIFT

(11) **EP 1 292 592 B1**
(45) Veröffentlichungstag und Bekanntmachung des Hinweises auf die Patenterteilung: **20.04.2011**
(21) Anmeldenummer: 01956484.8
(22) Anmeldetag: 19.06.2001
(51) Int. Cl.: C07D 401/12, C07D 417/12, A61K 31/4427, C07D 405/12, C07D 213/64

(54) **HETEROCYCLISCHE AMINOALKYLPYRIDINDERIVATE ALS PSYCHOPHARMAKA**
HETEROCYCLIC AMINOALKYL PYRIDINE DERIVATIVES AS PSYCHOPHARMACEUTICALS
DERIVES D'AMINOALKYLPYRIDINE HETEROCYCLIQUES UTILISES COMME MEDICAMENTS PSYCHOTROPES

(30) Priorität: 20.06.2000 DE 10029371
(43) Veröffentlichungstag der Anmeldung: 19.03.2003
(73) Patentinhaber: Merck Patent GmbH, 64293 Darmstadt (DE)
(72) Erfinder: DORSCH, Dieter, D-64372 Ober-Ramstadt (DE); BÖTTCHER, Henning, D-64287 Darmstadt (DE); ARLT, Michael, D-64665 ALSBACH (DE); GOTTSCHLICH, Rudolf, D-64354 Reinheim (DE); SEYFRIED, Christoph, D-64342 Seeheim-Jugenheim (DE); BARTOSZYK, Gerd, D-64331 Weiterstadt (DE); HARTING, Jürgen, D-64287 Darmstadt (DE)
(86) Internationale Anmeldenummer: PCT/EP2001/006915
(87) Internationale Veröffentlichungsnummer: WO 2001/098293

(56) Entgegenhaltungen:
- EP-A- 0 509 402
- EP-A- 0 707 007
- US-A- 4 983 586

## Beschreibung

Die Erfindung betrifft heterocyclische Aminoalkylpyridinderiviate, ihre Herstellung und ihre Verwendung als Psychopharmaka.

Die heterocyclischen Aminoalkylpyridinderiviate lassen sich darstellen durch die allgemeine Formel I 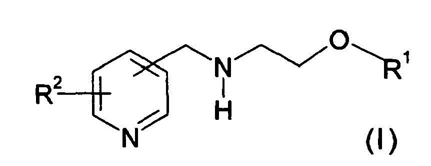 wobei
- R¹: das Radikal eines Heterocyclus mit 1 bis 3 Ringstrukturen, wobei je- de Ringstruktur gesättigt, ungesättigt oder aromatisch und gegebe- nenfalls mit anderen Ringstrukturen zu einem kondensierten Ringsys- tem anelliert ist und der Heterocyclus insgesamt 1 bis 4 N-, O- und/ oder S-Atome in den Ringstrukturen aufweist und gegebenenfalls einfach, zweifach oder dreifach substituiert ist durch eine oder meh- rere der Gruppen -A, -OR⁴, -N(R⁴)₂, -NO₂, -CN, Hal, -COOR⁴, - CON(R⁴)₂, -COR⁴, =O;
- R²: eine Phenylgruppe, die gegebenenfalls einfach, zweifach, dreifach, vierfach oder fünffach durch eine oder mehrere der Gruppen Hal, A, - O-A, -NO₂ oder -CN substituiert ist, bedeutet, oder eine Thienylgruppe, die gegebenenfalls einfach oder zweifach durch eine oder mehrere der Gruppen Hal, A, -O-A, -NO₂, -CN oder Thienyl substituiert ist, bedeutet;
- R⁴: H oder A;
- A: C₁-C₆-Alkyl, wobei gegebenenfalls 1 bis 7 Wasserstoffatome durch Fluor ersetzt sind;
- Hal: F, Cl, Br oder I;
sowie ihre verträglichen Salze und Solvate.

Psychosen, wozu auch Krankheiten aus dem Formkreis der Schizophrenie gehören, wurden auf eine Überaktivität des limbischen Dopaminsystems zurückgeführt (Snyder et al., Science 184: 1243-1253, 1974). Der antipsychotische Effekt von Neuroleptika wurde auf ihre D₂-antagonistischen Eigenschaften zurückgeführt (bezüglich der Nomenklatur der Rezeptoren: Basic Neurochemistry, Herausgeber: G. J. Siegel, B. W. Agranoff, R. W. Albers, P. B. Molinoff, 5. Auflage, Raven Press, Ltd., N. Y. USA, Kapitel 12 und 13; im übrigen folgende Fachaufsätze: Creese et al., Science 192: 481-483, 1976; Farde et al., Psychopharmacology 99: 28-31, 1989; Feeman et al., Nature 261: 717-719, 1976; Wiesel et al., Prog. Neuro-Psychopharmacol. & Biol. Psychiat. 14: 759-767, 1990). Folglich wurde die klassische Dopamin-Hypothese der Schizophrenie aufgestellt, nach der Neuroleptika an den D₂-Rezeptor zu binden haben. Der Einsatz klassischer D₂-Antagonisten ist aufgrund ihrer extrapyramidalen Nebenwirkungen vor allen Dingen bei chronischer Verabreichung stark eingeschränkt. Zu den extrapyramidalen Nebenwirkungen gehören z.B. Tremor, Akinesie, Dystonie und Akathisie (Cavallaro & Smeraldi, CNS Drugs 4: 278-293, 1995). Es gibt nur wenige Antipsychotika, die wesentlich weniger oder gar keine extrapyramidale Nebenwirkungen hervorrufen und die als "atypische Neuroleptika" bezeichnet werden (Kervin, Brit. J. Psychiatry 1964, 141-148, 1994). Das prototypische atypische Neuroleptikum Clozapin hat extrem geringe extrapyramidale Nebenwirkungen, verursacht aber andere gravierende Komplikationen wie die zuweilen tödliche Agranulocytose (Alvir et al., New Engl. J. Med. 329: 162-167, 1993).

Weil in Tieren 5-HT_{1A}-Agonisten antipsychotische Eigenschaften konventioneller Dopamin-D₂-Antagonisten verstärken (Wadenberg & Ahlenios, J. Neural. Transm. 74: 195-198, 1988) und die durch Dopamin-D₂-Antagonisten induzierte Katalepsie (Costall et al., Neuropharmacology 14: 859-868, 1975) verhindern, könnten 5-HT_{1A}-agonistische Eigenschaften von Vorteil sein. Die Wirksamkeit von Buspiron, ein Pharmakon mit 5-HT_{1A}-agonistischen und Dopamin-D₂-antagonistischen Eigenschaften, wurde bei Schizophrenie-Patienten nachgewiesen (Goff et al., J. Clin, Psychopharmacol. 11: 193-197, 1991). Außer verschiedenen Dopamin-Autorezeptoragonisten, die auch für den 5-HT_{1A}-Rezeptor eine signifikante Affinität aufweisen (z.B. U-86170F, Lahti et al., Naunyn-Schmiedeberg's Arch. Pharmacol. 344: 509-513, 1991), PD1431188 (Melzer et al., J. Pharmacol. Exp. Ther. 274: 912-920, 1995) und Roxindol (Bartoszyk et al., J. Pharmacol., Exp. Ther. 276: 41-48, 1996), wurden nur wenige Dopamin-D₂-Antagonisten entwickelt, die auch gegenüber dem 5-HT_{1A}-Rezeptor eine Affinität aufweisen wie Mazapertin (Reiz et al., J. Mid. Chem. 37: 1060-1062, 1994), S16924 (Millan et al., Br. J. Pharmacol. 114: 156 B, 1995) oder Ziprasidon (Seeger et al., J. Pharmacol. Exp. Ther. 275: 101-113, 1995). Diese bereits bekannten Verbindungen weisen im Hinblick auf Affinität oder Spezifität Nachteile auf. So zeigt Mazapertin auch eine Affinität für den α₁-Rezeptor. S16924 hat zusätzlich 5-HT_{2A/C}-antagonistische Eigenschaften und Ziprasidon bindet außerdem an die 5-HT_{1D/2A/2C}-Rezeptoren.

Es ist die Aufgabe der Erfindung Arzneimittel, insbesondere Psychopharmaka, bereitzustellen. Es ist eine weitere Aufgabe der Erfindung, Verbindungen bereitzustellen, die sowohl an den Dopamin-D₂-Rezeptor als auch an den 5-HT_{1A}-Rezeptor binden.

Diese Aufgabe wird gelöst durch die Verbindungen der allgemeinen Formel I und durch ihre verträglichen Salze und Solvate (siehe oben).

Ihre Bindungseigenschaften lassen sich durch bekannte 5-HT_{1A}-(Serotonin-)Bindungstest und Dopamin-Bindungstests bestimmen (5-HT_{1A}-(Serotonin-)Bindungstest: Matzen et al., J. Med. Chem., 43, 1149-1157, (2000) insbesondere Seite 1156 mit Verweis auf Eur. J. Pharmacol.: 140, 143-155 (1987); Dopamin-Bindungstests: Böttcher et al., J. Med. Chem.: 35, 4020-4026, (1992) mit Verweis auf J. Neurochem.: 46, 1058-1067 (1986)).

Diese Verbindungen unterscheiden sich sowohl von den vorgenannten atypischen Neuroleptika als auch von den aus EP-A 0 707 007 bekannten Amino(thio)ether der Formel 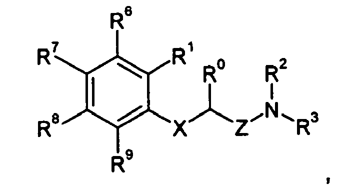 wobei R⁰ bis R⁹, X und Z die in EP-A 0 707 007 dargelegten Bedeutungen besitzen.

Die erfindungsgemäßen Verbindungen können zur Behandlung von Krankheiten eingesetzt werden, die mit dem Serotonin- und Dopamin-Neurotransmittersystem verbunden sind und bei denen hochaffine Serotoninrezeptoren (5-HT_{1A}-Rezeptoren) und/oder Dopamin-D₂-Rezeptoren beteiligt sind. Die wichtigste Indikation für die Verabreichung der Verbindung der allgemeinen Formel I sind Psychosen jeder Art, insbesondere auch Geisteskrankheiten aus dem Formenkreis der Schizophrenie. Darüber hinaus können die Verbindungen auch zur Verminderung von kognitiven Leistungsstörungen, also zur Verbesserung der Lernfähigkeit und des Gedächtnisses, eingesetzt werden. Auch zur Bekämpfung der Symptome von Morbus-Alzheimer sind die Verbindungen der allgemeinen Formel I geeignet. Darüber hinaus eignen sich die erfindungsgemäßen Substanzen der allgemeinen Formel I zur Prophylaxe und Kontrolle von Hirninfarkten (Apoplexia cerebri), wie Hirnschlag und zerebrale Ischemie. Ferner kommen die Substanzen zur Behandlung von Krankheiten wie krankhafte Angstzustände, Übererregung, Hyperaktivität und Aufmerksamkeitsstörungen bei Kindern und Jugendlichen, tiefgreifende Entwicklungstörungen und Störungen des Sozialverhaltens mit geistiger Retardierung, Depression, Zwangserkrankungen im engeren (OCD) und weiteren Sinne (OCSD) bestimmte sexuelle Funktionsstörungen, Schlafstörungen und Störungen in der Nahrungsaufnahme, sowie solcher Psychiatrischer Symptome in Rahmen der Altersdemenz und der Demenz vom Alzheimer-Typ, also Kranheiten des zentralen Nervensystems im weitesten Sinn, in Frage.

Die Verbindungen der allgemeinen Formel I und ihre verträglichen Salze und Solvate können also als aktive Inhaltsstoffe für Arzneimittel wie Anxiolytika, Antidepressiva, Neuroleptika und/oder Antihypertensiva eingesetzt werden.
- R¹: stellt einen gegebenenfalls substituierten Heterozyklus mit 1- bis 3- Ringstrukturen dar. Die Anzahl der Ringstrukturen eines Heterozyklus' ist mit der Anzahl der Ringöffnungen, die gedanklich ausgeführt werden müssen, um den Heterozyklus in eine acyclische Verbindung zu über- führen, identisch. Die Ringstrukturen können unabhängig voneinander, soweit das chemisch möglich ist, gesättigt, ungesättigt oder aromatisch sein. Eine Ringstruktur kann gegebenenfalls mit anderen Ringstrukturen zu einem kondensierten Ringsystem anelliert sein. Auch nicht- aromatische gesättigte oder ungesättigte Ringstrukturen können in Ana- logie zu kondensierten Ringsystemen miteinander verbunden sein, das heißt Bindungen miteinander teilen, wie dies zum Beispiel bei Steroiden oder bei Chroman der Fall ist. Der Heterozyklus umfaßt insgesamt 1 bis 4 Stickstoff, Sauerstoff und/oder Schwefel-Atome, welche in den Ring- strukturen die Kohlenstoffatome ersetzen. Vorzugsweise sind diese N-, O- und/oder S-Atome nicht benachbart. Der Heterozyklus ist gegebe- nenfalls einfach, zweifach oder dreifach durch eine oder mehrere der Gruppen -A, -OR⁴, -N (R⁴)₂, -NO₂, -CN, Hal, -COOR⁴, -CON(R⁴)₂. - COR⁴, =O substituiert. R¹ bedeutet bevorzugt Chinolyl, Isochinolyl, die gegebenenfalls mindestens einen Chlor-Substituenten aufweisen, oder Indolyl, Benzothiazolyl, Cumaronyl, Cumarinyl, Pyridyl oder Carbazolyl, wobei gegebenenfalls mindestens ein Wasserstoff des Chinolyls, Iso- chinolyls, Indolyls oder Benzothiazolyls durch eine Methylgruppe oder Ethoxycarbonylgruppe ersetzt ist. Insbesondere trägt R¹ die Bedeutung Chinolin-7-yl, Chinolin-8-yl, wobei gegebenenfalls an Position 5 ein Wasserstoff durch ein Chloratom ersetzt ist, oder Indol-4-yl oder Indol- 7-yl, wobei gegebenenfalls ein Wasserstoff an Position 2 des Chinolyls oder Indolyls durch eine Methylgruppe oder Ethoxycarbonylgruppe er- setzt ist. Besonders bevorzugt sind die Bedeutungen Indol-4-yl, 2- Methylindol-4-yl und Chinolin-8-yl für R¹.
- R²: stellt eine Phenylgruppe dar, die gegebenenfalls einfach, zweifach, drei- fach, vierfach oder fünffach durch eine oder mehrere Gruppen Hal, -A, -O-A-, -NO₂ oder -CN substituiert ist. R² kann ferner die Bedeutung ei- ner Thienyl-Gruppe tragen, die gegebenenfalls einfach oder zweifach durch eine oder mehrere der Gruppen Hal, -A, -O-A-, NO₂, -CN oder Thienyl substituiert ist. R² bedeutet vorzugsweise Fluorphenyl, Diflu- orphenyl, Cyanophenyl oder Tolyl. Insbesondere hat R² die Bedeutung Fluorphenyl, 2,4-Difluorphenyl, 3-Cyanophenyl oder 4-Methylphenyl.
- R⁴: trägt die Bedeutung H oder -A, wobei H bevorzugt ist.
- A: bedeutet C₁-C₆-Alkyl, wobei gegebenenfalls 1 bis 7 Wasserstoffatome durch Fluor ersetzt sind. A kann verzweigt oder unverzweigt sein und ist bevorzugt Methyl, Ethyl, Propyl, Isopropyl, n-Butyl, sec-Butyl, tert-Butyl ferner auch Pentyl, 1-, 2- oder 3-Methylbutyl, 1,1-, 1,2- oder 2,2- Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1- Ethyl-1-methylpropyl, 1-Ethyl-2-methylpropyl, 1,1,2- oder 1,2,2- Trimethylpropyl. Besonders bevorzugt für A ist Methyl, Ethyl, Isopropyl, n-Propyl, n-Butyl oder tert-Butyl.
- Hal: bedeutet F, Cl, Br oder I, wobei F und Cl, insbesondere F, bevorzugt sind.

Die Substituenten R¹, R² können unabhängig voneinander eine der vorgenannten Bedeutungen annehmen. Soweit in R¹, R² die Bedeutung eines die Begriffe R⁴, A oder Hal aufweisenden Substituenten annimmt, so können R⁴, A und Hal in jedem Substituenten R¹, R² eine andere Bedeutung aufweisen. Die Verbindungen der allgemeinen Formel I sind umso stärker bevorzugt, je mehr ihrer Substituenten bevorzugte Bedeutungen aufweisen und je stärker diese Bedeutungen bevorzugt sind.

Eine bevorzugte Verbindung der allgemeinen Formel I wird durch die allgemeine Formel VIII beschrieben, wobei der Substituent -CH₂-NH-CH₂-CH₂-O-R¹ in meta-Stellung zum Pyridinstickstoff steht:

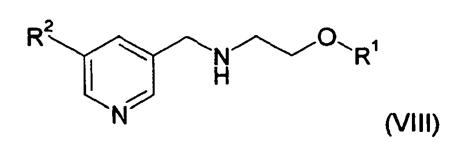

Soweit die Verbindungen der allgemeinen Formel I optisch aktiv sind, umfaßt die Formel I sowohl jede isolierte optische Antipode als auch die entsprechenden gegebenenfalls racemischen Gemische in jeder denkbaren Zusammensetzung.

Eine Verbindung der allgemeinen Formel I kann mit einer Säure in das entsprechende Salz (das heißt Säureadditionssalz) überführt werden. Säuren die verträgliche (das heißt biokompatible und ausreichend bioverfügbare) Salze ergeben, sind für diese Reaktion geeignet. Es ist also möglich, anorganische Säuren wie Schwefelsäure oder Halogenwasserstoffsäuren wie Salzsäure, Bromsäure oder Phosphorsäuren wie Orthophosphorsäure, Salpetersäure, Sulfaminsäure, aliphatische, alizyklische, araliphatische, aromatische oder heterocyclische monobasische oder polybasische Carbonsäuren, Sulfonsäuren oder Schwefelsäurederivate wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salizylsäure, 2-Phenylpropionsäure, Zitronensäure, Glukonsäure, Ascorbinsäure, Nikotinsäure, Isonikotinsäure, Methansulfonsäure oder Ethansulfonsäure, Ethandisulfonsäure, 2-Hydroxyethansulfonsäure, Benzolsulfonsäure, Parataluolsulfonsäure, Naphthalinmonosulfonsäure und Naphthalindisulfonsäure und Schwefelsäurelaurylester zu verwenden.

Falls erwünscht, können die korrespondierenden freien Basen der allgemeinen Formel I durch die Behandlung ihrer Salze mit starken Basen wie Natriumhydroxid, Kaliumhydroxid oder Natrium- oder Kaliumcarbonat, freigesetzt werden, vorausgesetzt, daß sich keine anderen sauren Gruppen in dem Molekül befinden. In den letztgenannten Fällen, in welchen die Verbindungen der allgemeinen Formel I freie saure Gruppen tragen, kann Salzbildung auch durch Behandlung mit starken Basen bewirkt werden. Geeignete Basen sind Alkalimetallhydroxyde, Erdalkalihydroxyde oder organische Basen in Form von primären, sekundären oder tertiären Aminen.

Unter Solvaten der Verbindungen der allgeimeinen Formel I werden Anlagerungen von chemisch "inerten" Lösungsmittelmolekülen an die Verbindungen der Formel I verstanden, die sich aufgrund ihrer gegenseitigen Anziehungskraft ausbilden. Solvate sind zum Beispiel Mono- und Dihydrate oder Additionsverbindungen mit Alkoholen wie Methanol oder Ethanol.

Es ist bekannt, daß Pharmaka synthetisch in Derivate umgewandelt werden können (zum Beispiel in Alkyl- oder Acylderivate, in Zucker- oder Oligopeptidderivate und andere), die im Körper metabolisch durch extrazelluläre oder intrazelluläre Enzyme in die aktiven Verbindungen der allgemeinen Formel I zurückverwandelt werden. Die Erfindung bezieht sich auch auf solche "Pro-Drug-Derivate" der Verbindungen der allgemeinen Formel I.

Ein weiterer Gegenstand der Erfindung ist die Verwendung einer Verbindung der allgemeinen Formel I oder eines ihrer verträglichen Salze oder Solvate zur Herstellung eines Arzneimittels, welches sich zur Behandlung von menschlichen oder tierischen Krankheiten, insbesondere von Krankheiten des zentralen Nervensystems wie krankhafte Spannungszustände, Depression und/oder Psychosen, zur Verminderung von Nebenwirkungen bei der Behandlung von Bluthochdruck (z.B. mit α-Methyldopa), zur Behandlung von endokrinologischen und/oder gynäkologischen Krankheiten, z.B. zur Behandlung von Akromegalie, Hypogonadismus, sekundärer Amenorrhoe, dem postmenstruellen Syndrom und unerwünschter Laktation in der Pubertät und zur Prophylaxe und Therapie von zerebralen Krankheiten (z.B. von Migräne) insbesondere in der Geriatrie in ähnlicher Weise wie bestimmte Ergot-Alkaloide und zur Kontrolle und Prophylaxe von Hirninfarkt (Apoplexia cerebri) wie Hirnschlag und zerebraler Ischaemie eignet. Darüber hinaus sind die pharmazeutischen Zubereitungen und Arzneimittel, die eine Verbindung der allgemeinen Formel I enthalten, zur Verbesserung des kognitiven Leistungsvermögens und zur Behandlung von Morbus-Alzheimer-Symptomen geeignet. Insbesondere eignen sich solche Arzneimittel zur Behandlung von Geisteskrankheiten aus dem Formenkreis der Schizophrenie und zur Bekämpfung von psychotischen Angstzuständen. Der Begriff Behandlung schließt im Rahmen der Erfindung Prophylaxe und Therapie menschlicher oder tierischer Kranheiten ein.

Die Substanzen der allgemeinen Formel I werden normalerweise analog zu bekannten, kommerziell erhältlichen pharmazeutischen Zubereitungen (z.B. von Bromocriptin und Dihydroergocornin), vorzugsweise in Dosen von zwischen 0,2 und 500 mg, insbesondere von zwischen 0,2 und 15 mg pro Dosiseinheit verabreicht. Die tägliche Dosiseinheit ist zwischen 0,001 und 10 mg pro kg Körpergewicht. Geringe Dosen (von zwischen 0,2 und 1 mg pro Dosiseinheit, 0,001 bis 0,005 mg pro kg Körpergewicht) sind besonders geeignet für pharmazeutische Zubereitungen zur Behandlung von Migräne. Eine Dosis zwischen 10 und 50 mg pro Dosiseinheit wird für andere Indikationen bevorzugt. Allerdings hängt die zu verabreichende Dosis von einer Vielzahl von Faktoren ab, z.B. von der Wirksamkeit der entsprechenden Komponente, dem Alter, dem Körpergewicht und der allgemeinen Verfassung des Patienten.

Ein weiterer Gegenstand der Erfindung ist ein Verfahren zur Herstellung einer pharmazeutischen Zubereitung, das die Überführung einer Verbindung der allgemeinen Formel I oder eines ihrer verträglichen Salze oder Solvate zusammen mit einem geeigneten Träger in eine geeignete Dosierungsform beinhaltet. Die Verbindungen der allgemeinen Formel I können zusammen mit wenigstens einem Träger oder Hilfsstoff, gegebenenfalls in Kombination mit einem weiteren aktiven Inhaltsstoff, in eine geeignete Dosierungsform gebracht werden.

Geeignete Träger sind organische oder anorganische Substanzen, die für die enterale (z.B. orale) oder parenterale oder topische Verabreichung geeignet sind und die mit den erfindungsgemäßen Substanzen der allgemeinen Formel I nicht reagieren. Beispiele solcher Träger sind Wasser, Gemüseöle, Benzylalkohole, Polyethylenglykole, Gelatine, Kohlenhydrate wie Lactose und Stärke, Magnesiumstearat, Talk und Rohvaseline. Tabletten, beschichtete Tabletten, Kapseln, Sirupe, Säfte, Tropfen oder Zäpfchen werden insbesondere für die enterale Verabreichung eingesetzt. Lösungen, vorzugsweise ölige oder wässrige Lösungen, wie Suspensionen, Emulsionen oder auch Implantate werden für die parenterale Verabreichung verwandt. Salben, Creme oder Puder kommen bei der äußerlichen Anwendung zum Einsatz. Die Verbindungen der allgemeinen Formel I können auch lyophilisiert und die resultierenden Lyophilisate zu injizierbaren Paparationen verarbeitet werden.

Gegenstand der Erfindung sind weiter Arzneimittel, die mindestens eine Verbindung der allgemeinen Formel I bzw. eines ihrer verträglichen Salze oder Solvate enthalten und gegebenenfalls weitere Inhaltsstoffe wie Träger, Hilfsstoffe usw. Diese Zubereitungen können als Arzneimittel zur Behandlung von menschlichen oder tierischen Krankheiten eingesetzt werden.

Die vorgenannten Arzneimittel können sterilisiert und zusammen mit Hilfsstoffen wie Gleitmitteln, Konservierungsmitteln, Stabilisatoren und/oder Befeuchtungsmitteln, Emulgatoren osmotisch wirksamen Substanzen, Puffern, Farbstoffen oder Geschmacksverbesserern zu anderen pharmazeutischen Präparaten verarbeitet werden.

Ein weiterer Gegenstand der Erfindung ist ein Verfahren zur Herstellung der Verbindungen der allgemeinen Formel I. Dieses Verfahren eignet sich für Verbindungen, bei denen der Substituent -CH₂-NH-CH₂-CH₂-O-R¹ in meta-Stellung zum Stickstoff des Pyridinrings steht.

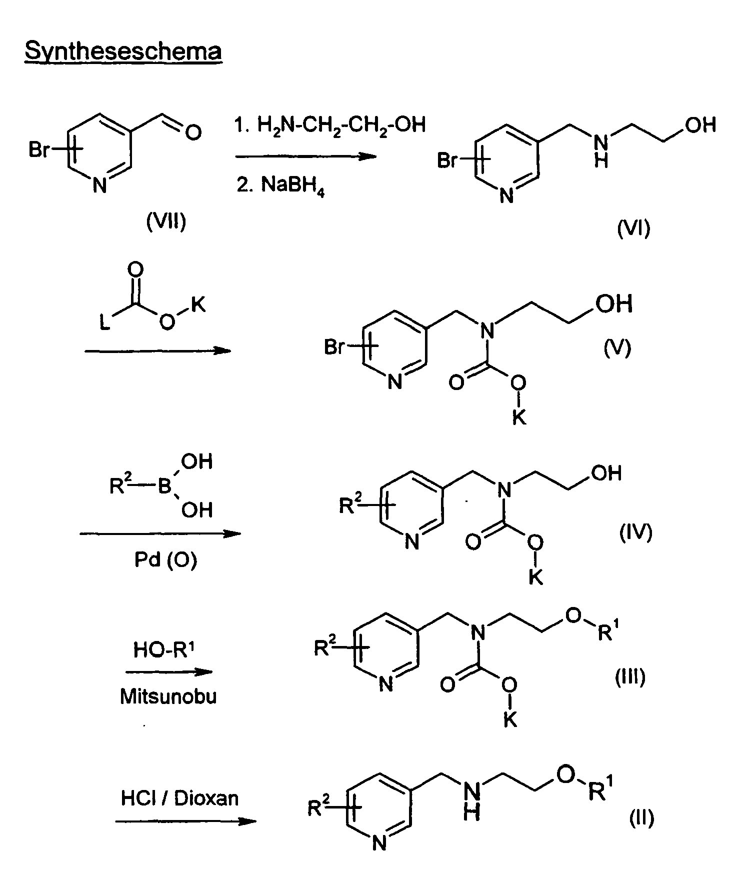

In einem ersten Schritt wird Brompyridin-3-yl-methanal (VII) mit H₂N-CH₂-CH₂-OH und z.B. Natriumborhydrid zu einer Verbindung der allgemeinen Formel VI umgesetzt. In einem zweiten Schritt wird das sekundäre Amin der allgemeinen Formel VI mit einer Schutzgruppe versehen. Dies erfolgt unter Zusatz von Reagenzien der Formel L-CO-O-K, wobei K die Bedeutung von *tert-*butyl, Fluoren-9-yl-methyl oder Benzyl hat und L entweder Cl, N₃ oder *tert-*butoxycarbonyloxy darstellt. Verbindungen der allgemeinen Formel V werden unter Einsatz eines entsprechenden Pd(0)-Katalysators mit geeigneten Boronsäure-Derivaten (R²-B(OH)₂) umgesetzt, wobei Verbindungen der Formel IV entstehen, bei welchen das Brom-Atom durch den R²-Substituenten ausgetauscht ist (Suzuki-Kopplung, vgl. A. Suzuki, N. Miyaura Chem. Rev. 1995, 95, 2457-2483; A.R.Martin, Y. Yang, Acta Chem. Scand. 1993, 47, 221-230). Mit Hilfe der Mitsunobu-Reaktion werden Verbindungen der allgemeinen Formel IV mit Verbindungen der Formel HO-R¹ umgesetzt, wobei Verbindungen der Formel III entstehen. Details über die Namensreaktion lassen sich entnehmen aus: Mitsunobu, Bull. Chem. Soc. Jpn.: 40, 4235-4238 (1967); Hughes, Org. React.: 42, 335-656 (1992); Mitsunobu, Synthesis, 1-28 (1981); Hughes et al., J. Am. Chem. Soc.: 110, 6487-6491 (1988); Crich et al., J. Org. Chem.: 54, 257 - 259 (1989); Camp et al., J. Org. Chem.: 54, 3045-3049, 3049-3054 (1989). Anschließend wird die Schutzgruppe aus Verbindungen der allgemeinen Formel III entfernt, wobei Verbindungen der allgemeinen Formel II entstehen. Alternativ erfolgt die Synthese über folgende Zwischenstufen:

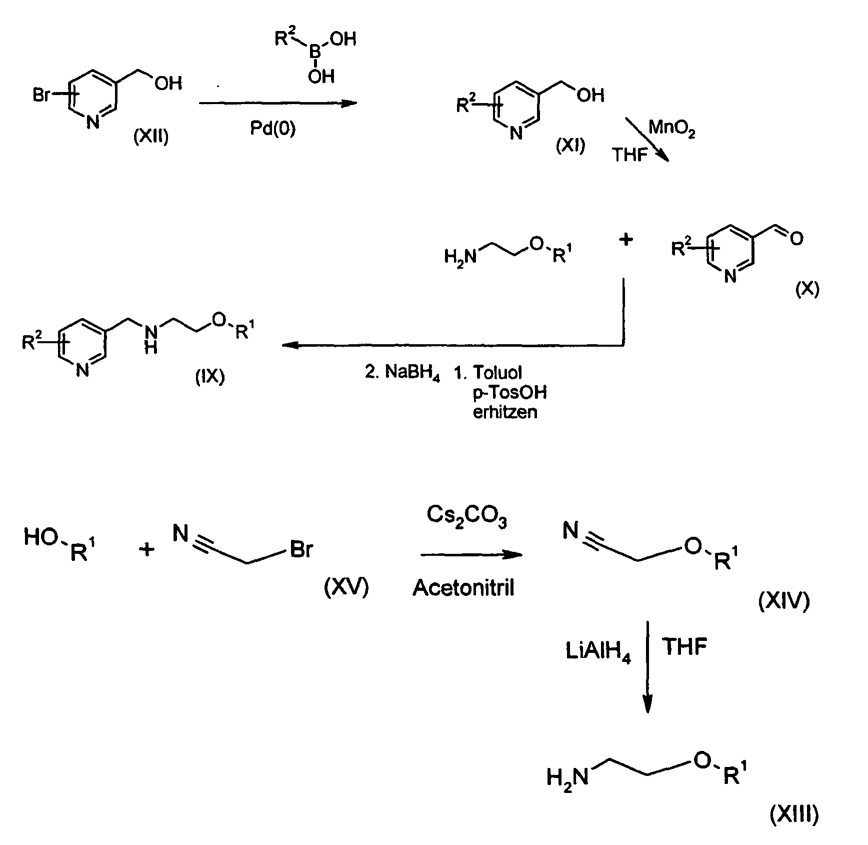

Dieses Alternatiwerfahren ist ebenfalls Gegenstand der Erfindung.

Durch die Suzuki-Reaktion wird ein Brompyridylmethanol der Formel XII mit einem Boronsäurederivat R²-B(OH)₂ unter Pd(0)-Katalyse gekoppelt (Suzuki-Kopplung, vgl. A. Suzuki, N. Miyaura Chem. Rev. 1995, 95, 2457-2483; A.R.Martin, Y. Yang, Acta Chem. Scand. 1993, 47, 221-230). Der dabei entstehende Alkohol der Formel XI wird z.B. mit MnO₂ in Tetrahydrofuran zum korrespondierenden Aldehyd der Formel X oxidiert. Dieser wird mit einem Amin (R¹-O-CH₂-CH₂-NH₂) unter Bildung einer Schiff sehen Base umgesetzt, die sogleich zum korrepondierenden sekundären Amin reduziert wird.

Das Amin kann auf verschiedenen Wegen hergestellt werden. In einer bevorzugten Ausführungsform ist es mit Aminen der allgemeinen Formel XIII identisch. Das Amin der Formel XIII wird durch Alkylierung eines Alkohols R¹-OH mit einem Nitril der Formel XV unter Bildung eines Nitrils der Formel XIV und anschließender Reduktion zum korrespondierenden primären Amin gebildet.

Ein weiterer Gegenstand der Erfindung sind Verbindungen der allgemeinen Formeln II, III, IV, V und VI, wobei die Substituenten die bereits genannten Bedeutungen besitzen.
Ein weiterer Gegenstand der Erfindung sind Verbindungen der allgemeinen Formel IX, wobei die Substituenten die bereits genannten Bedeutungen besitzen.

Die Erfindung wird durch die nachfolgenden Beispiele beschrieben.
Das Molekulargewicht (M+H⁺) wird mit Hilfe von Elektronenspray-Ionisationsmassenspektroskopie bestimmt. Die massenspektroskopischen Daten stammen aus HPLC/MS-Läufen (HPLC gekoppelt mit einem Elektrospray-lonisationsmassenspektrometer). Die Zahlenwerte sind, wie bei diesem Verfahren üblich, nicht die Molekulargewichte der unveränderten Verbindungen, sondern die Molekulargewichte der protonierten Verbindungen (im folgenden: M+H⁺). Die Methode ist in folgenden Literaturstellen beschrieben: M. Yamashita, J. B. Fenn, J. Phys. Chem. 88, 1984, 4451-4459; C. K. Meng et al., Zeitschrift für Physik D 10, 1988, 361-368; J. B. Fenn et al., Science 246, 1989, 64-71.

### Beispiel 1

### Herstellung der Verbindung 5-[(3-Fluor-phenyl)-pyridin-3-yl-methyl]-[2-(2-Methyl-1-H-indol-4-yloxy)-ethyl]-amindihydrochlorid.

Die Synthese folgt dem Syntheseschema wobei das Bromatom der Formel VII in Metastellung zum Pyridinstickstoff steht, R² 3-Fluorphenyl bedeutet, Y Ethylen darstellt, X die Bedeutung von Sauerstoff hat und R¹ 2-Methylindol-4-yl darstellt.

Eine Lösung von 113 g (607 mmol) 5-Brom-pyridin-3-carbaldehyd, 41.9 ml (700 mmol) Ethanolamin und 12 g (10 mmol)Toluol-4-sulfonsäure-Monohydrat in 1 I Toluol wird 5 Stunden am Wasserabscheider erhitzt. Nach dem Erkalten werden zur Lösung 500 ml Methanol zugefügt und unter Rühren portionsweise78.8 g (2.00 mol) Natriumborhydrid eingetragen. Das Reaktionsgemisch wird 18 Stunden bei Raumtemperatur gerührt, im Vakuum eingeengt und der Rückstand zwischen Wasser und Ethylacetat verteilt. Die organische Phase wird eingedampft: 2-[(5-Bromo-pyridin-3-ylmethyl)-amino]-ethanol ((M+H⁺) = 231, 233) als leicht gelbliches Öl, das ohne weitere Reinigung für die nächste Reaktion eingesetzt wird.

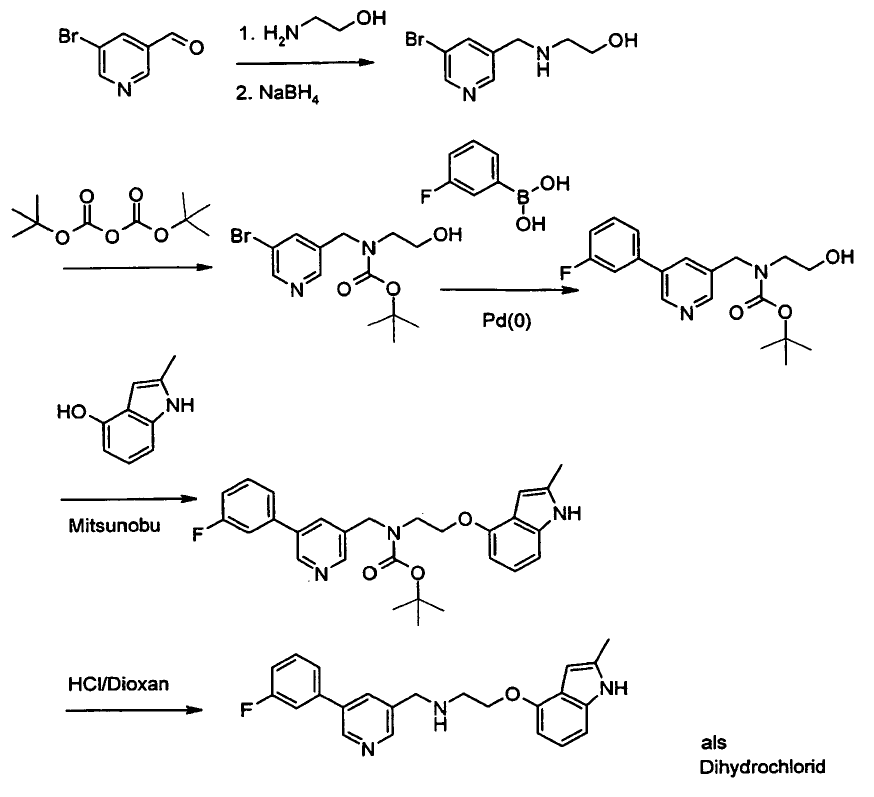

Zu einer auf 0°C gekühlten Lösung von 120 g (ca. 519 mmol) rohem 2-[(5-Bromo-pyridin-3-ylmethyl)-amino]-ethanol in 1 I Dichlormethan wird eine Lösung von 164 g (750 mmol) Di-tert-butyldicarbonat in 1 I Dichlormethan langsam zugetropft. Das Reaktionsgemisch wird 18 Stunden bei Raumtemperatur gerührt, mit 3 I Wasser versetzt und die organische Phase abgetrennt. Die organische Phase wird über Natriumsulfat getrocknet, eingeengt und der Rückstand an einer Kieselgelsäule mit Dichlormethan/Methanol 9:1 chromatographiert: (5-Brom-pyridin-3-ylmethyl)-(2-hydroxyethyl)-carbaminsäure-tert-butylester ((M+H⁺) = 331, 333) als gelbliches Öl.

Eine Lösung von 5.40 g (16.3 mmol) (5-Brom-pyridin-3-ylmethyl)-(2-hydroxyethyl)-carbaminsäure-tert-butylester, 4.6 g (32.9 mmol) 3-Fluorbenzolboronsäure, 250 g (0.27 mmol) Tris-(dibenzylidenaceton)-dipalladium(0) und 220 mg (1.09 mmol) Tri-tert-butyl-phosphin in 200 ml Dioxan wird mit 10.6 g (32.5 mmol) Caesiumcarbonat versetzt und 4 Stunden bei 100°C gerührt. Das Reaktionsgemisch wird auf Wasser gegeben und mit Ethylacetat extrahiert. Die organische Phase wird eingedampft und der Rückstand an einer Kieselgelsäule mit Petrolether/Ethylacetat als Laufmittel chromatographiert: [5-(3-Fluorphenyl)-pyridin-3-ylmethyl]-(2-hydroxyethyl)-carbaminsäure-tert-butylester ((M+H⁺) = 347) als farbloses Öl.

Eine Lösung von 100 mg (0.289 mmol) [5-(3-Fluorphenyl)-pyridin-3-ylmethyl]-(2-hydroxyethyl)-carbaminsäure-tert-butylester und 86.8 mg (0.590 mmol) 4-Hydroxy-2-methylindol in 5 ml THF wird mit 240 mg polymergebundenem Triphenylphosphin versetzt und 10 Minuten bei Raumtemperatur gerührt. Dann werden 166 mg (720 mmol) Di-tert-butylazodicarboxylat zugegeben und 18 Stunden bei Raumtemperatur gerührt. Anschließend wird 1 g stark basischer lonenaustauscher zugegeben, 1 Stunde gerührt und filtriert. Das Filtrat wird eingedampft. Es wird erhalten: [5-(3-Fluor-phenyl)-pyridin-3-ylmethyl]-[2-(2-methyl-1*H-*indol-4-yloxy)-ethyl]-carbaminsäure-tert-butylester ((M+H⁺) = 476) als gelbliches Öl.

Der im vorigen Beispiel erhaltene rohe [5-(3-Fluor-phenyl)-pyridin-3-ylmethyl]-[2-(2-methyl-1*H-*indol-4-yloxy)-ethyl]-carbaminsäure-tert-butylester wird mit 2 ml 4N Chlorwasserstoff in Dioxan versetzt, mit 1 ml Methanol verdünnt und 2 Stunden bei Raumtemperatur gerührt. Das Reaktionsgemisch wird eingedampft, der Rückstand mit Diethylether verrührt und filtriert. Der Rückstand wird zwischen 1 N Natronlauge und Ethylacetat verteilt, die organische Phase eingedampft und der Rückstand mit einem Überschuss 0.1 N HCl in Isopropanol versetzt. Das Lösungsmittel wird abdestilliert: 5-(3-Fluor-phenyl)-pyridin-3-ylmethyl]-[2-(2-methyl-1*H-*indol-4-yloxy)-ethyl]-amin Dihydrochlorid als bräunlicher Feststoff, (M+H⁺) = 376.

### Beispiel 2:

### Herstellung der Verbindung 5-(4-Fluorphenyl)-pyridin-3-ylmethyl]-[2-(1H-indol-4-yloxy)-ethyl]-aminhydrochlorid nach einer alternativen Synthesemethode.

Eine Lösung von 244 g (1.30 mol) (5-Brompyridin-3-yl)-methanol und 200 g (1.43 mol) 4-Fluorbenzolboronsäure in 1.5l Toluol wird mit 750 ml Wasser, 168 g (2.00mol) Natriumhydrogencarbonat und 5.0 g (4.3 mmol) Tetrakis(triphenylphosphin)-palladium(0) versetzt und 24 Stunden zum Sieden erhitzt. Die organische Phase wird abgetrennt, über Natriumsulfat getrocknet und eingedampft. Der Rückstand wird aus tert-Butylmethylether umkristallisiert: [5-(4-Fluorphenyl)-pyridin-3-yl]-methanol als farblose Kristalle vom Schmpkt. 71-72°C.

Eine Lösung von 62.0 g (305 mmol) [5-(4-Fluorphenyl)-pyridin-3-yl]-methanol in 500 ml THF wird mit 135 g (1.554 mol) Mangandioxid versetzt und 18 Stunden bei 45°C gerührt. Das Reaktionsgemisch wird filtriert und das Filtrat eingedampft. Der Rückstand wird aus tert.-Butylmethylether umkristallisiert: 5-(4-Fluorphenyl)-pyridin-3-carbaldehyd als farblose Kristalle; (M+H⁺) 202.

Eine Lösung von 1.00 g (7.51 mmol) 4-Hydroxyindol und 965 mg (7.80 mmol) Bromacetonitril in 30 ml Acetonitril wird mit 2.6 g (8.0 mmol) Caesiumcarbonat versetzt und 18 Stunden bei Raumtemperatur gerührt. Das Reaktionsgemisch wird filtriert und das Filtrat eingedampft: (1*H-*Indol-4-yloxy)-acetonitril als grauer Feststoff; (M+H⁺): 173.

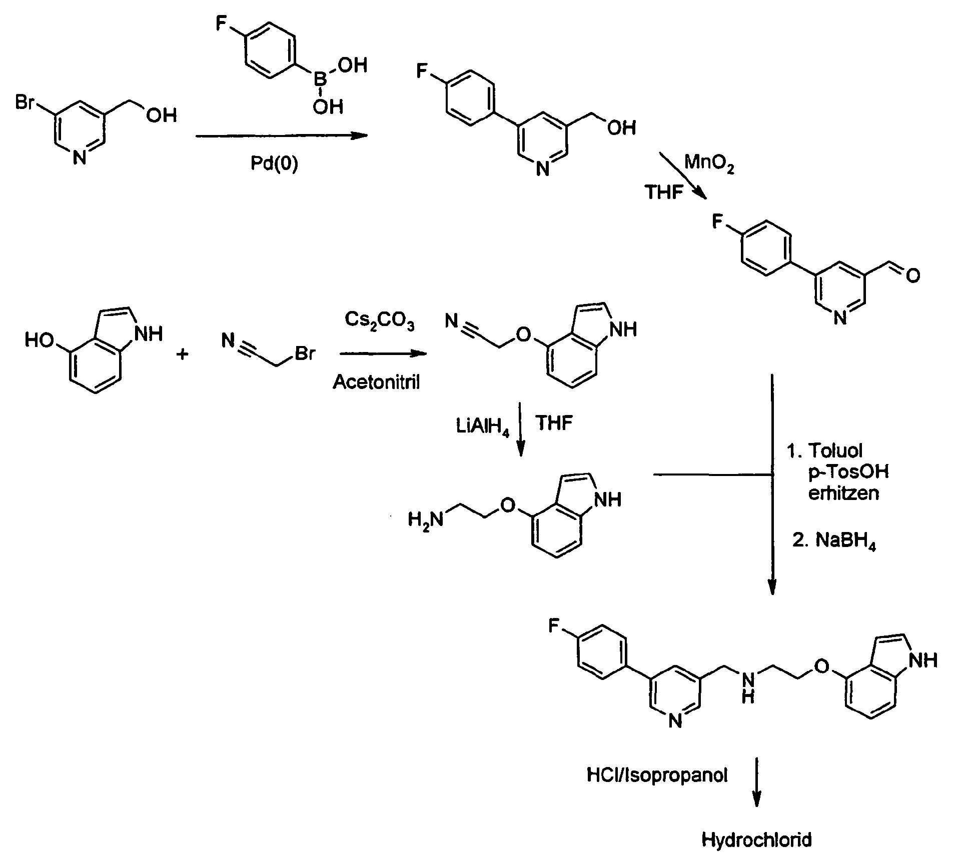

Eine auf 0°C gekühlte Lösung von 1.20 g (6.97 mmol) (1*H-*Indol-4-yloxy)-acetonitril in 20 ml THF wird mit 531 mg (14.0 mmol) Lithiumaluminiumhydrid versetzt und unter Rühren auf Raumtemperatur erwärmt. Nach 2 Stunden Rühren bei Raumtemperatur wird das Reaktionsgemisch mit 2 g mit Wasser angefeuchtetem Natriumsulfat versetzt und filtriert. Das Filtrat wird eingedampft: 2-(1*H-*Indol-4-yloxy)-ethylamin als gelbliches Öl; (M+H⁺): 177.

Zu einer Lösung von 805 mg (4.00 mmol) 5-(4-Fluorphenyl)-pyridin-3-carbaldehyd in 80 ml Toluol werden 700 mg (3.972 mmol) 2-(1*H-*Indol-4-yloxy)-ethylamin und 100 mg (0.52 mmol) Toluol-4-Sulfonsäure-Monohydrat gegeben und das Gemisch 18 Stunden am Wasserabscheider zum Sieden erhitzt. Nach dem Erkalten wird das Gemisch mit 50 ml Methanol versetzt und 630 mg (16.0 mmol) Natriumborhydrid zugegeben. Das Reaktionsgemisch wird 18 Stunden bei Raumtemperatur gerührt, im Vakuum eingeengt und der Rückstand zwischen Wasser und Ethylacetat verteilt. Die organische Phase wird eingedampft und der Rückstand an einer Kieselgelsäule mit Ethylacetat/Ethanol 8:2 als Laufmittel chromatograpiert: [5-(4-Fluorphenyl)-pyridin-3-ylmethyl]-[2-(1*H-*indol-4-yloxy)-ethyl]-amin als farblose Kristalle; (M+H⁺) 362. Das so erhaltene Produkt wird mit 3 ml 0.1 N HCl in Isopropanol versetzt und eine Stunde bei Raumtemperatur gerührt. Das Lösungsmittel wird abdestilliert, der Rückstand mit Diethylether versetzt und filtriert: [5-(4-Fluorphenyl)-pyridin-3-ylmethyl]-[2-(1*H-*indol-4-yloxy)-ethyl]-amin Hydrochlorid als bräunliche Kristalle; (M+H⁺) 362.

### Beispiel 3:

Die folgenden Verbindungen werden analog hergestellt.

| **Nr.** | **Struktur** | **Name bzw. Salzform** | **(M+ H⁺)** |
|---|---|---|---|
| a | 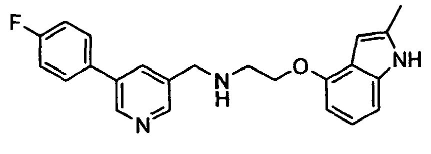 | [5-(4-Fluorphenyl)-pyridin-3-ylmethyl]-[2-(2-methyl-1*H-*indol-4-yloxy)-ethyl]-aminhydrochlorid | 376 |
| b | 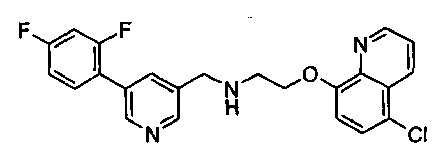 | [2-(5-Chlorchinolin-8-yloxy)-ethyl]-[5-(2,4-difluorphenyl)-pyridin-3-ylmethyl]-aminhydrochlorid | 426 |
| c | 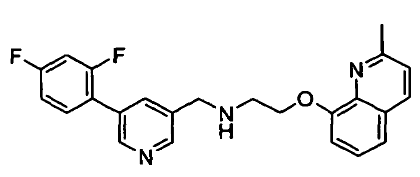 | [5-(2,4-Difluorphenyl)-pyridin-3-ylmethyl]-[2-(2-methylchinolin-8-yloxy)-ethyl]-aminhydrochlorid | 406 |
| d | 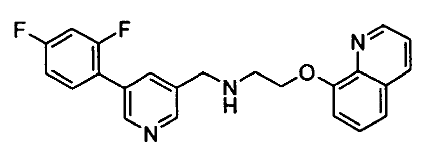 | [5-(2,4-Difluorphenyl)-pyridin-3-ylmethyl]-[2-(chinolin-8-yloxy)-ethyl]-aminhydrochlorid | 392 |
| e | 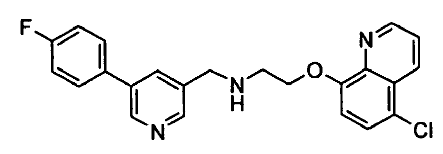 | [2-(5-Chlorchinolin-8-yloxy)-ethyl]-[5-(4-fluorphenyl)-pyridin-3-ylmethyl]-aminhydrochlorid | 408 |
| f | 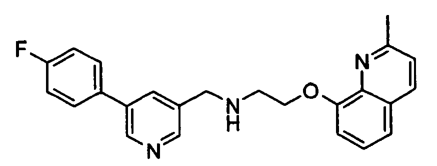 | [5-(4-Fluorphenyl)-pyridin-3-ylmethyl]-[2-(2-methylchinolin-8-yloxy)-ethyl]-aminhydrochlorid | 388 |
| g | 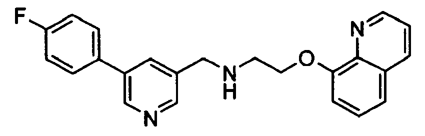 | [5-(4-Fluorphenyl)-pyridin-3-ylmethyl]-[2-(chinolin-8-yloxy)-ethyl]-aminhydrochlorid | 374 |
| h | 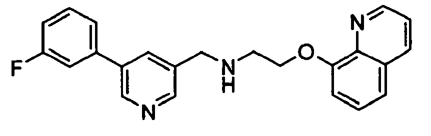 | [5-(3-Fluorphenyl)-pyridin-3-ylmethyl]-[2-(chinolin-8-yloxy)-ethyl]-aminhydrochlorid | 374 |
| i | 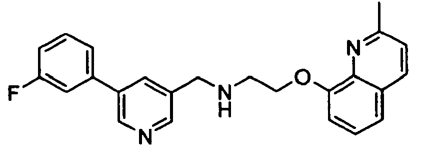 | [5-(3-Fluorphenyl)-pyridin-3-ylmethyl]-[2-(2-methylchinolin-8-yloxy)-ethyl]-aminhydrochlorid | 388 |
| j | 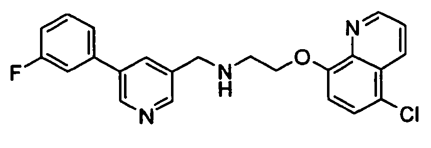 | [2-(5-Chlorchinolin-8-yloxy)-ethyl]-[5-(3-fluorphenyl)-pyridin-3-ylmethyl]-aminhydrochlorid | 408 |
| k | 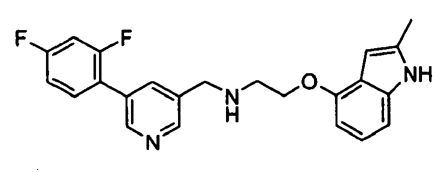 | [5-(2,4-Difluorphenyl)-pyridin-3-ylmethyl]-[2-(2-methyl-1*H-*indol-4-yloxy)-ethyl]-aminhydrochlorid | 394 |
| l | 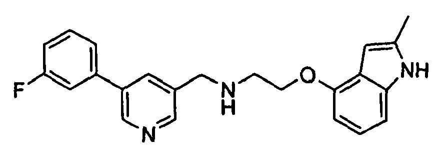 | [5-(3-Fluorphenyl)-pyridin-3-ylmethyl]-[2-(2-methyl-1*H-*indol-4-yloxy)-ethyl]-aminhydrochlorid | 376 |
| m | 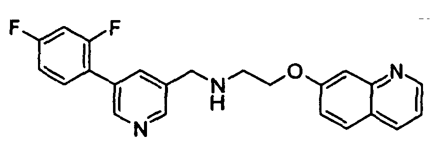 | [5-(2,4-Difluorphenyl)-pyridin-3-ylmethyl]-[2-(chinolin-7-yloxy)-ethyl]-aminhydrochlorid | 392 |
| n | 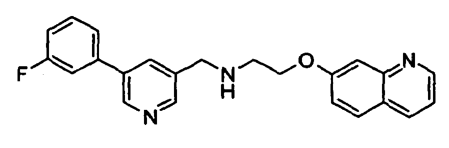 | [5-(3-Fluorphenyl)-pyridin-3-ylmethyl]-[2-(chinolin-7-yloxy)-ethyl]-aminhydrochlorid | 374 |
| o | 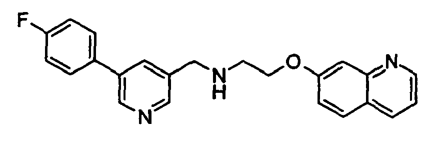 | [5-(4-Fluorphenyl)-pyridin-3-ylmethyl]-[2-(chinolin-7-yloxy)-ethyl]-aminhydrochlorid | 374 |
| p | 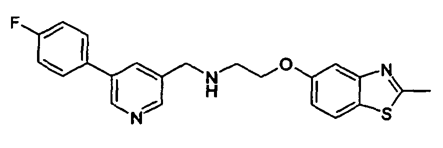 | [5-(4-Fluorphenyl)-pyridin-3-ylmethyl]-[2-(2-methyl-benzothiazol-5-yloxy)-ethyl]-amindihydrochlorid | 394 |
| q | 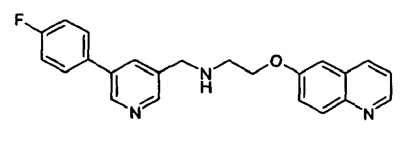 | [5-(4-Fluorphenyl)-pyridin-3-ylmethyl]-[2-(chinolin-6-yloxy)-ethyl]-amintrihydrochlorid | 374 |
| r | 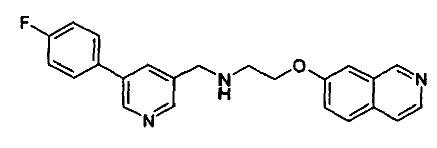 | [5-(4-Fluorphenyl)-pyridin-3-ylmethyl]-[2-(isochinolin-7-yloxy)-ethyl]-amin-trihydrochlorid | 374 |
| s | 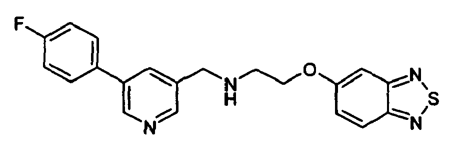 | [2-(Benzo[1,2,5]thia-diazol-5-yloxy)-ethyl]-[5-(4-fluorphenyl)-pyridin-3-ylmethyl]-amin-dihydrochlorid | 381 |
| t | 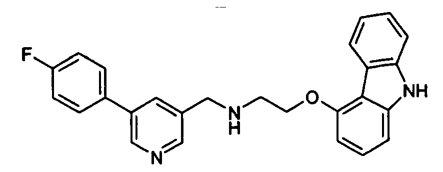 | [2-(9*H-*Carbazol-4-yloxy)-ethyl]-[5-(4-fluor-phenyl)-pyridin-3-ylmethyl]-amin-dihydrochlorid | 412 |
| u | 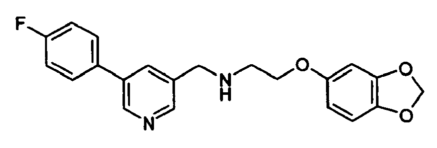 | [2-(Benzo[1,3]dioxol-5-yloxy)-ethyl]-[5-(4-fluorphenyl)-pyridin-3-yl-methyl]-amindi-hydrochlorid | 367 |
| v | 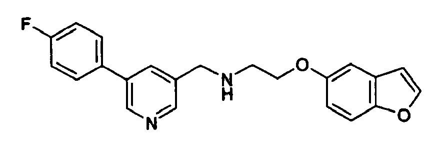 | [2-(Benzofuran-5-yloxy)-ethyl]-[5-(4-fluorphenyl)-pyridin-3-ylmethyl]-amin-dihydrochlorid | 363 |
| w | 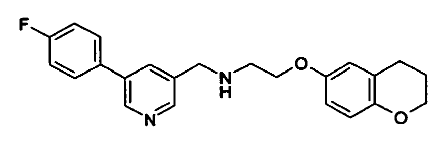 | [2-(Chroman-6-yloxy)-ethyl]-[5-(4-fluorphenyl)-pyridin-3-ylmethyl]-amin dihydrochlorid | 448 |
| x | 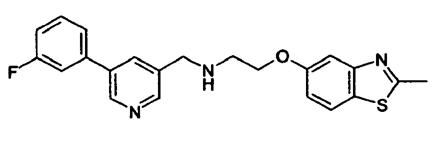 | [5-(3-Fluorphenyl)-pyridin-3-ylmethyl]-[2-(2-methylbenzothiazol-5-yloxy)-ethyl]-amindihydrochlorid | 394 |
| y | 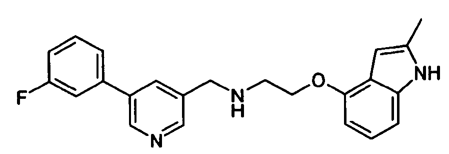 | [5-(3-Fluorphenyl)-pyridin-3-ylmethyl]-[2-(2-methyl-1*H-*indol-4-yloxy)-ethyl]-amin dihydrochlorid | 376 |
| z | 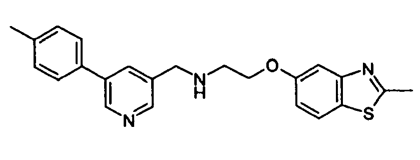 | [2-(2-Methyl-benzothiazol-5-yloxy)-ethyl]-(5-p-tolyl-pyridin-3-ylmethyl)-amin-dihydrochlorid | 390 |
| aa | 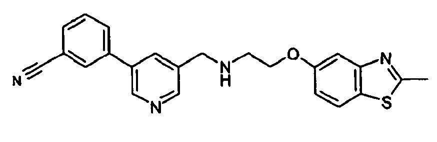 | 3-(5-{[2-(2-Methyl-benzothiazol-5-yloxy)-ethylamino]-methyl}-pyridin-3-yl)-benzynitril-hydrochlorid | 401 |
| bb | 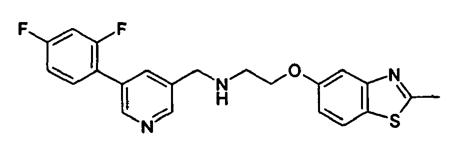 | [5-(2,4-Difluorphenyl)-pyridin-3-ylmethyl]-[2-(2-methylbenzothiazol-5-yloxy)-ethyl]-amindihydrochlorid | 412 |
| cc | 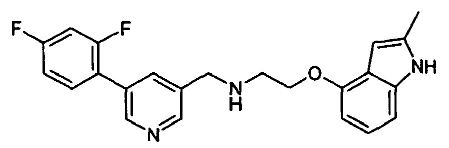 | [5-(2,4-Difluorphenyl)-pyridin-3-ylmethyl]-[2-(2-methyl-1*H-*indol-4-yloxy)-ethyl]-amin-dihydrochlorid | 394 |
| dd | 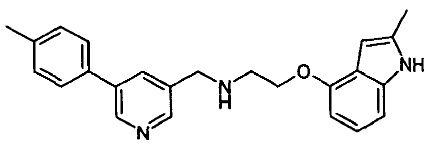 | [2-(2-Methyl-1*H-*indol-4-yloxy)-ethyl]-(5-p-tolyl-pyridin-3-ylmethyl)-amindihydrochlorid | 372 |
| ee | 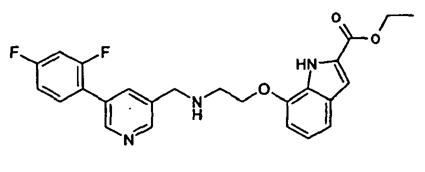 | 7-(2-{[5-(2,4-Difluor-phenyl)-pyridin-3-ylmethyl]-amino}-ethoxy)-1*H-*indol-2-carbonsäureethyl-esterdihydrochlorid | 452 |
| ff | 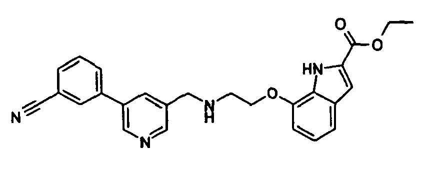 | 7-(2-{[5-(3-Cyano-phenyl)-pyridin-3-ylmethyl]-amino}-ethoxy)-1*H-*indol-2-carbonsäureethylester-dihydrochlorid | 441 |
| gg | 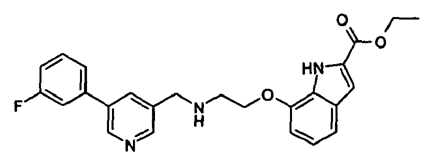 | 7-(2-{[5-(3-Fluorphenyl)-pyridin-3-ylmethyl]-amino}-ethoxy)-1*H-*indol-2-carbonsäureethylester-dihydrochlorid | 434 |
| hh | 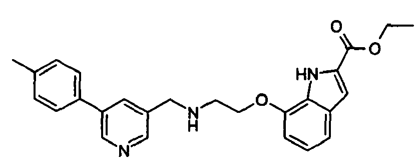 | 7-{2-[(5-p-Tolyl-pyridin-3-ylmethyl)-amino]-ethoxy}-1*H-*indol-2-carbonsäure-ethylesterdihydrochlorid | 413 |
| ii | 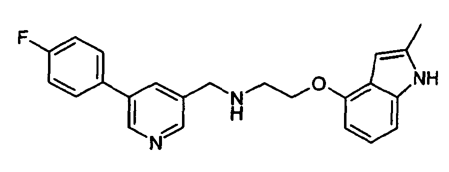 | [5-(4-Fluorphenyl)-pyridin-3-ylmethyl]-[2-(2-methyl-1*H-*indol-4-yloxy)-ethyl]-amin-dihydrochlorid | 376 |
| jj | 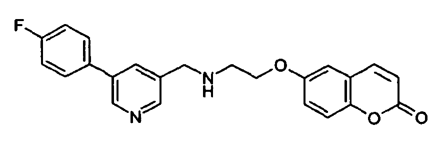 | 6-(2-{[5-(4-Fluorphenyl)-pyridin-3-ylmethyl]-amino}-ethoxy)-chromen-2-on-dihydrochlorid | 391 |
| kk | 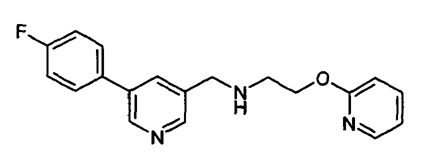 | [5-(4-Fluorphenyl)-pyridin-3-ylmethyl]-[2-(pyridin-2-yloxy)-ethyl]-amin-trihydrochlorid | 324 |
| ll | 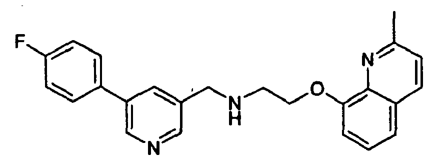 | [5-(4-Fluorphenyl)-pyridin-3-ylmethyl]-[2-(2-methylchinolin-8-yloxy)-ethyl]-amintrihydro-chlorid | 388 |
| mm | 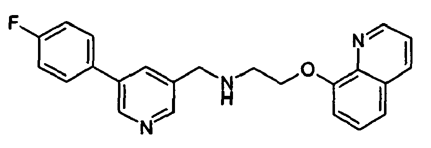 | [5-(4-Fluorphenyl)-pyridin-3-ylmethyl]-[2-(chinolin-8-yloxy)-ethyl]-amin-trihydrochlorid | 374 |
| nn | 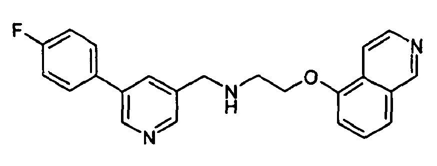 | [5-(4-Fluorphenyl)-pyridin-3-ylmethyl]-[2-(isochinolin-5-yloxy)-ethyl]-amintrihydro-hlorid | 374 |
| oo | 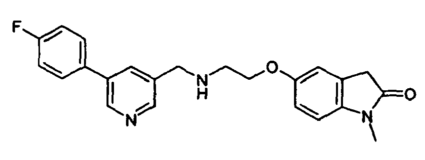 | 5-(2-{[5-(4-Fluorphenyl)-pyridin-3-ylmethyl]-amino}-ethoxy)-1-methyl-1,3-dihydroindol-2-on-dihydrochlorid | 392 |
| pp | 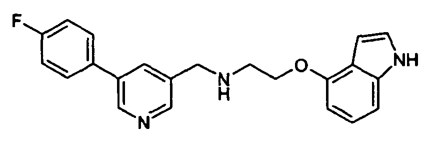 | [5-(4-Fluorphenyl)-pyridin-3-ylmethyl]-[2-(1*H-*indol-4-yloxy)-ethyl]-amin-hydrochlorid | 362 |
| qq | 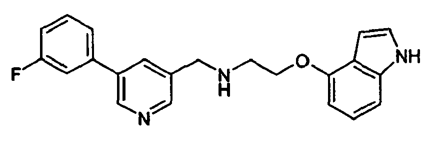 | [5-(3-Fluorphenyl)-pyridin-3-ylmethyl]-[2-(1*H-*indol-4-yloxy)-ethyl]-amin-hydrochlorid | 362 |
| rr | 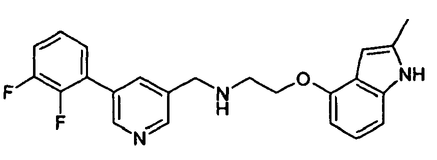 | [5-(2,3-Difluorphenyl)-pyridin-3-ylmethyl]-[2-(2-methyl-1*H-*indol-4-yloxy)-ethyl]-amin-hydrochlorid | 394 |
| ss | 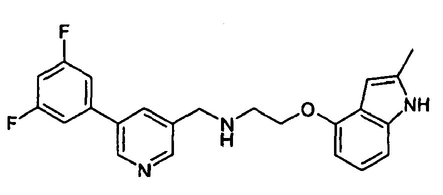 | [5-(3,5-Difluorphenyl)-pyridin-3-ylmethyl]-[2-(2-methyl-1*H-*indol-4-yloxy)-ethyl]-amin-hydrochlorid | 394 |
| tt | 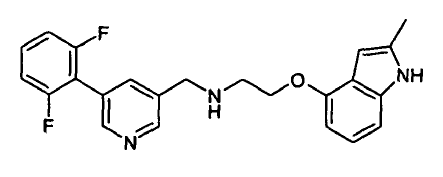 | [5-(2,6-Difluorphenyl)-pyridin-3-ylmethyl]-[2-(2-methyl-1*H-*indol-4-yloxy)-ethyl]-amin-hydrochlorid | 394 |
| uu | 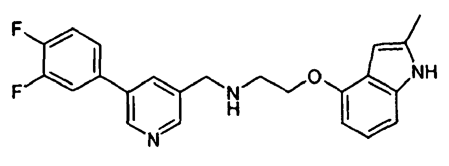 | [5-(3,4-Difluorphenyl)-pyridin-3-ylmethyl]-[2-(2-methyl-1*H*-indol-4-yloxy)-ethyl]-amin-hydrochlorid | 394 |
| vv | 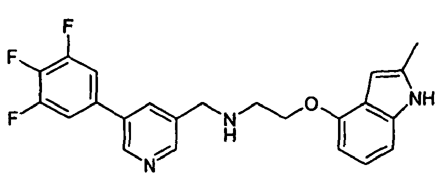 | [2-(2-Methyl-1*H-*indol-4-yloxy)-ethyl]-[5-(3,4,5-trifluorphenyl)-pyridin-3-ylmethyl]-aminhydrochlorid | 412 |
| ww | 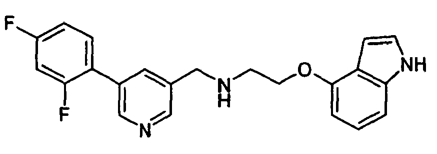 | [5-(2,4-Difluoro-phenyl)-pyridin-3-ylmethyl]-[2-(1*H-*indol-4-yloxy)-ethyl]-aminhydrochlorid | 380 |
| xx | 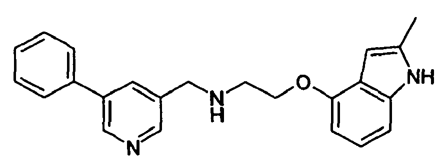 | [2-(2-Methyl-1*H-*indol-4-yloxy)-ethyl]-(5-phenyl-pyridin-3-ylmethyl)-aminhydrochlorid | 358 |
| yy | 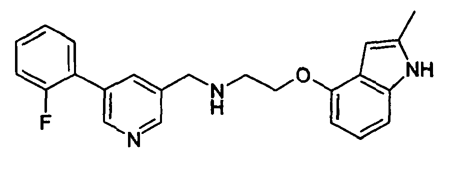 | [5-(2-Fluorphenyl)-pyridin-3-ylmethyl]-[2-(2-methyl-1*H-*indol-4-yloxy)-ethyl]-aminhydro-chlorid | 376 |
| zz | 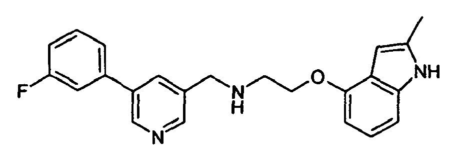 | [5-(3-Fluorphenyl)-pyridin-3-ylmethyl]-[2-(2-methyl-1*H-*indol-4-yloxy)-ethyl]-amin-Methansulfonat | 376 |
| aaa | 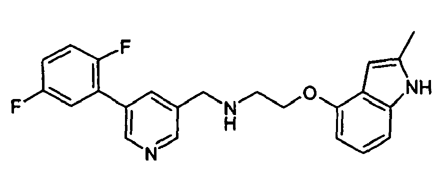 | [5-(2,5-Difluorphenyl)-pyridin-3-ylmethyl]-[2-(2-methyl-1*H-*indol-4-yloxy)-ethyl]-amin-hydrochlorid | 394 |
| bbb | 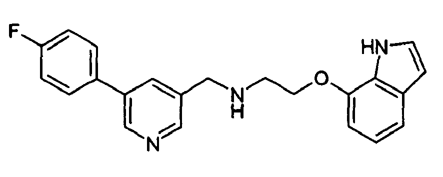 | [5-(4-Fluorphenyl)-pyridin-3-ylmethyl]-[2-(1*H*-indol-7-yloxy)-ethyl]-amin-hydrochlorid | 362 |
| ccc | 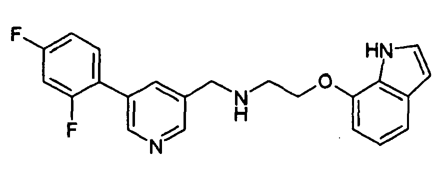 | [5-(2,4-Difluorphenyl)-pyridin-3-ylmethyl]-[2-(1*H-*indol-7-yloxy)-ethyl]-amin-hydrochlorid | 380 |
| ddd | 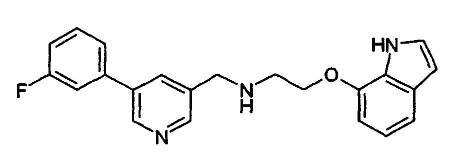 | [5-(3-Fluorphenyl)-pyridin-3-ylmethyl]-[2-(1*H-*indol-7-yloxy)-ethyl]-amin-hydrochlorid | 362 |
| eee | 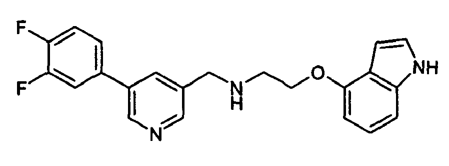 | [5-(3,4-Difluorphenyl)-pyridin-3-ylmethyl]-[2-(1*H-*indol-4-yloxy)-ethyl]-amin-hydrochlorid | 380 |
| fff | 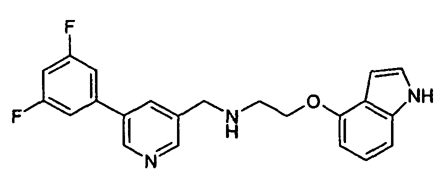 | [5-(3,5-Difluorphenyl)-pyridin-3-ylmethyl]-[2-(1*H-*indol-4-yloxy)-ethyl]-amin-hydrochlorid | 380 |
| ggg | 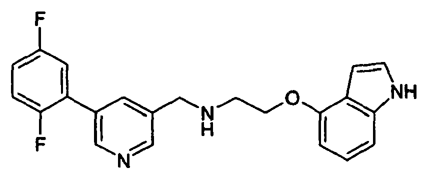 | [5-(2,5-Difluorphenyl)-pyridin-3-ylmethyl]-[2-(1*H-*indol-4-yloxy)-ethyl]-amin-hydrochlorid | 380 |
| hhh | 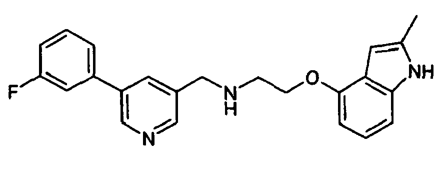 | [5-(3-Fluorphenyl)-pyridin-3-ylmethyl]-[2-(2-methyl-1*H-*indol-4-yloxy)-ethyl]-amin-Malonat | 376 |
| iii | 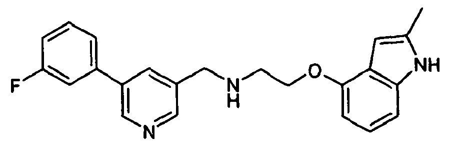 | [5-(3-Fluorphenyl)-pyridin-3-ylmethyl]-[2-(2-methyl-1*H-*indol-4-yloxy)-ethyl]-amin-Hemifumarat | 376 |
| jjj | 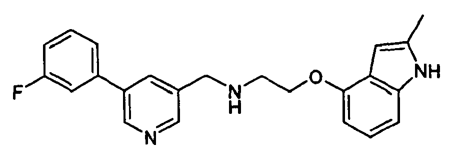 | [5-(3-Fluorphenyl)-pyridin-3-ylmethyl]-[2-(2-methyl-1*H-*indol-4-yloxy)-ethyl]-amin-Hemisulfat | 376 |
| kkk | 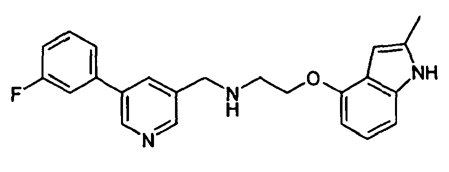 | [5-(3-Fluorphenyl)-pyridin-3-ylmethyl]-[2-(2-methyl-1*H-*indol-4-yloxy)-ethyl]-amin-Tartrat | 376 |
| lll | 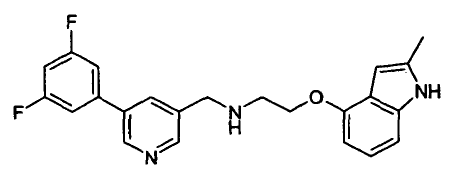 | [5-(3,5-Difluorphenyl)-pyridin-3-ylmethyl]-[2-(2-methyl-1*H-*indol-4-yloxy)-ethyl]-amin-Hemifumarat | 394 |
| mmm | 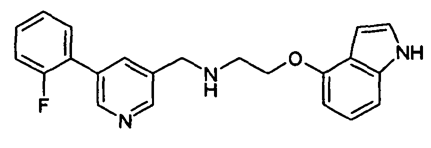 | [5-(2-Fluorphenyl)-pyridin-3-ylmethyl]-[2-(1*H-*indol-4-yloxy)-ethyl]-amin-dihydrochlorid | 362 |
| nnn | 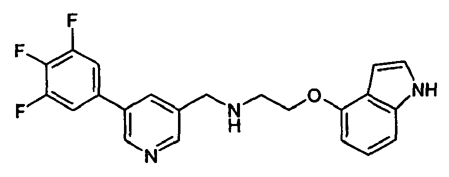 | [2-(1*H-*Indol-4-yloxy)-ethyl]-[5-(3,4,5-trifluorphenyl)-pyridin-3-ylmethyl]-amin-dihydrochlorid | 398 |
| ooo | 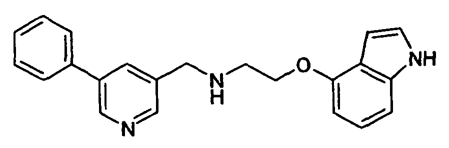 | [2-(1*H-*Indol-4-yloxy)-ethyl]-(5-phenyl-pyridin-3-ylmethyl)-amin-dihydrochlorid | 344 |

### Beispiel 4:

### Ampullen zur Injektion

Eine Lösung von 100 g einer Verbindung der allgemeinen Formel I und 5 g Dinatriumhydrogenphosphat wird in 3 I doppelt destilliertem Wasser auf pH 6,5 mit 2 N Salzsäure eingestellt, steril filtriert und in Injektionsampullen gefüllt, und lyrphilisiert. Dabei wurden sterile Bedingungen eingehalten. Jede Injektionsampulle enthält 5 mg der aktiven Komponente der allgemeinen Formel I.

### Beispiel 5:

Eine Mischung von 20 g einer Verbindung der allgemeinen Formel I wird unter Erwärmung mit 100 g Soja-Lecithin und 1400 g Kakaobutter gemischt und in Vertiefungen gegossen. Jedes Zäpfchen enthält 20 mg der aktiven Komponente.

### Beispiel 6:

Eine Lösung, enthaltend 1 g einer Verbindung der allgemeinen Formel I, 9,38 g NaH₂PO₄ x 2 H₂O, 28,48 g Na₂HPO₄ x 12 H₂O und 0,1 g Benzalkoniumchlorid, wird mit 940 ml zweifach destilliertem Wasser angesetzt. Die Lösung wird auf pH 6,8 eingestellt auf einen Liter mit zweifach destilliertem Wasser aufgefüllt und durch Strahlung sterilisiert. Diese Lösung kann in Form von Augentropfen benutzt werden.

### Beispiel 7:

### Salbe

500 mg einer Verbindung der allgemeinen Formel I werden mit 99,5 g Rohvaseline unter aseptischen Bedingungen vermengt.

### Beispiel 8:

### Tabletten

100 g einer Verbindung der allgemeinen Formel I, 1 kg Lactose, 600 g mikrokristalline Zellulose, 600 g Maisstärke, 100 g Polyvinylpyrrolidon, 80 g Talk und 10 g Magnesiumsterat werden gemischt und in üblicher Weise zu Tabletten gepreßt, so daß eine Tablette 100 mg der aktiven Komponente enthält.

### Beispiel 9:

### Beschichtete Tabletten

Tabletten werden wie in Beispiel 7 hergestellt und anschließend auf bekannte Weise mit Saccharose, Maisstärke, Talk, Tragantgummi und Farbstoffen beschichtet.

### Beispiel 10:

### Kapseln

Hartgelatinekapseln werden mit einer Verbindung der allgemeinen Formel I auf bekannte Art und Weise gefüllt, so daß jede Kapsel 5 mg der aktiven Komponente enthält.

### Beispiel 11:

### lnhalierungsspray

14 g einer Verbindung der allgemeinen Formel I werden in 10 I isotonischer Kochsalzlösung aufgelöst. Die Lösung wird in kommerziell erhältliche Spraycontainer, die einen Pumpmechanismus besitzen, eingefüllt. Die Lösung kann in den Mund oder in die Nase gesprüht werden. Ein Sprüh-Stoß (ungefähr 0,1 ml) entspricht einer Dosis von 0,14 mg einer Verbindung der allgemeinen Formel I.

## Patentansprüche

1. Verbindung der allgemeinen Formel I 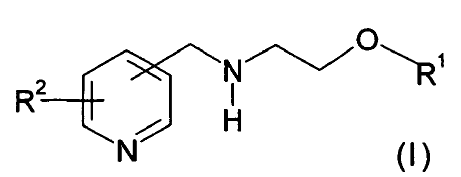 wobei
R¹ das Radikal eines Heterocyclus mit 1 bis 3 Ringstrukturen, wobei je- de Ringstruktur gesättigt, ungesättigt oder aromatisch und gegebe- nenfalls mit anderen Ringstrukturen zu einem kondensierten Ringsys- tem anelliert ist und der Heterocyclus insgesamt 1 bis 4 N-, O- und/ oder S-Atome in den Ringstrukturen aufweist und gegebenenfalls einfach, zweifach oder dreifach substituiert ist durch eine oder meh- rere der Gruppen -A, -OR⁴, -N(R⁴)₂, -NO₂, -CN, Hal, -COOR⁴, - CON(R⁴)_{2,} -COR⁴, =O;
R² eine Phenylgruppe, die gegebenenfalls einfach, zweifach, dreifach, vierfach oder fünffach durch eine oder mehrere der Gruppen Hal, A, - O-A, -NO₂ oder -CN substituiert ist, bedeutet, oder eine Thienylgruppe, die gegebenenfalls einfach oder zweifach durch eine oder mehrere der Gruppen Hal, A, -O-A, -NO₂, -CN oder Thienyl substituiert ist, bedeutet;
R⁴ H oder A;
A C₁-C₆-Alkyl, wobei gegebenenfalls 1 bis 7 Wasserstoffatome durch Fluor ersetzt sind;
Hal F, Cl, Br oder I;
sowie ihre verträglichen Salze und Solvate.

2. Verbindung nach Anspruch 1, **dadurch gekennzeichnet, daß** der Substituent -CH₂-NH-CH₂-CH₂-O-R¹ in meta-Stellung zum Stickstoff des Pyridinrings steht.

3. Verbindung nach einem der Anspruch 1 oder 2, **dadurch gekennzeichnet, daß** R² in meta-Stellung zum Stickstoff des mit R² verbundenen Pyridinrings steht.

4. Verbindung nach einem der Ansprüche 1 bis 3, **gekennzeichnet durch** mindestens eines der folgenden Merkmale:
R¹ bedeutet Chinolyl, Isochinolyl, die gegebenenfalls mindestens einen Chlor-Substituenten aufweisen, oder Indolyl, Benzothiazolyl, Cuma- ronyl, Cumarinyl, Pyridyl oder Carbazolyl, wobei gegebenenfalls min- destens ein Wasserstoff des Chinolyls, Isochinolyls, Indolyls oder Benzothiazolyls **durch** eine Methylgruppe oder Ethoxycarbonylgruppe ersetzt ist;
R² bedeutet Fluorphenyl, Difluorphenyl, Cyanophenyl oder Tolyl.

5. Verbindung nach einem der Ansprüche 1 bis 4, **gekennzeichnet durch** mindestens eines der folgenden Merkmale:
R¹ bedeutet Chinolin-7-yl, Chinolin-8-yl, wobei gegebenenfalls an Positi- on 5 ein Wasserstoff **durch** ein Chlor-Atom ersetzt ist, oder Indol-4-yl oder Indol-7-yl, wobei gegebenenfalls ein Wasserstoff an Position 2 des Chinolyls oder Indolyls **durch** eine Methylgruppe oder Ethoxycar- bonylgruppe ersetzt ist;
R² bedeutet Fluorphenyl, 2,4-Difluorphenyl, 3-Cyanophenyl oder 4- Methylphenyl.

6. Verbindung nach Anspruch 1, ausgewählt aus
a) [5-(4-Fluorphenyl)-pyridin-3-ylmethyl]-[2-(2-methyl-1*H-*indol-4-yloxy)-ethyl]-aminhydrochlorid
b) [2-(5-Chlorchinolin-8-yloxy)-ethyl]-[5-(2,4-difluorphenyl)-pyridin-3-ylmethyl]-aminhydrochlorid
c) [5-(2,4-Difluorphenyl)-pyridin-3-ylmethyl]-[2-(2-methylchinolin-8-yloxy)-ethyl]-aminhydrochlorid
d) [5-(2,4-Difluorphenyl)-pyridin-3-ylmethyl]-[2-(chinolin-8-yloxy)-ethyl]-aminhydrochlorid
e) [2-(5-Chlorchinolin-8-yloxy)-ethyl]-[5-(4-fluorphenl)-pyridin-3-ylmethyl]-aminhydrochlorid
f) [5-(4-Fluorphenyl)-pyridin-3-ylmethyl]-[2-(2-methylchinolin-8-yloxy)-ethyl]-aminhydrochlorid
g) [5-(4-Fluorphenyl)-pyridin-3-ylmethyl]-[2-(chinolin-8-yloxy)-ethyl]-aminhydrochlorid
h) [5-(3-Fluorphenyl)-pyridin-3-ylmethyl]-[2-(chinolin-8-yloxy)-ethyl]-aminhydrochlorid
i) [5-(3-Fluorphenyl)-pyridin-3-ylmethyl]-[2-(2-methylchinolin-8-yloxy)-ethyl]-aminhydrochlorid
j) [2-(5-Chlorchinolin-8-yloxy)-ethyl]-[5-(3-fluorphenyl)-pyridin-3-ylmethyl]-aminhydrochlorid
k) [5-(2,4-Difluorphenyl)-pyridin-3-ylmethyl]-[2-(2-methyl-1*H-*indol-4-yloxy)-ethyl]-aminhydrochlorid
l) [5-(3-Fluorphenyl)-pyridin-3-ylmethyl]-[2-(2-methyl-1*H-*indol-4-yloxy)-ethyl]-aminhydrochlorid
m) [5-(2,4-Difluorphenyl)-pyridin-3-ylmethyl]-[2-(chinolin-7-yloxy)-ethyl]-aminhydrochlorid
n) [5-(3-Fluorphenyl)-pyridin-3-ylmethyl]-[2-(chinolin-7-yloxy)-ethyl]-aminhydrochlorid
o) [5-(4-Fluorphenyl)-pyridin-3-ylmethyl]-[2-(chinolin-7-yloxy)-ethyl]-aminhydrochlorid
p) [5-(4-Fluorphenyl)-pyridin-3-ylmethyl]-[2-(2-methyl-benzothiazol-5-yloxy)-ethyl]-amindihydrochlorid
q) [5-(4-Fluorphenyl)-pyridin-3-ylmethyl]-[2-(chinolin-6-yloxy)-ethyl]-amintrihydrochlorid
r) [5-(4-Fluorphenyl)-pyridin-3-ylmethyl]-[2-(isochinolin-7-yloxy)-ethyl]-amintrihydrochlorid
s) [2-(Benzo[1,2,5]thiadiazol-5-yloxy)-ethyl]-[5-(4-fluorphenyl)-pyridin-3-ylmethyl]-amindihydrochlorid
t) [2-(9*H-*Carbazol-4-yloxy)-ethyl]-[5-(4-fluorphenyl)-pyridin-3-ylmethyl]-amindihydrochlorid
u) [2-(Benzo[1,3]dioxol-5-yloxy)-ethyl]-[5-(4-fluorphenyl)-pyridin-3-ylmethyl]-amindihydrochlorid
v) [2-(Benzofuran-5-yloxy)-ethyl]-[5-(4-fluorphenyl)-pyridin-3-ylmethyl]-amindihydrochlorid
w) [2-(Chroman-6-yloxy)-ethyl]-[5-(4-fluorphenyl)-pyridin-3-ylmethyl]-amindihydrochlorid
x) [5-(3-Fluorphenyl)-pyridin-3-ylmethyl]-[2-(2-methylbenzothiazol-5-yloxy)-ethyl]-amindihydrochlorid
y) [5-(3-Fluorphenyl)-pyridin-3-ylmethyl]-[2-(2-methyl-1*H-*indol-4-yloxy)-ethyl]-amindihydrochlorid
z) [2-(2-Methyl-benzothiazol-5-yloxy)-ethyl]-(5-p-tolyl-pyridin-3-ylmethyl)-amindihydrochlorid
aa) 3-(5-{[2-(2-Methyl-benzothiazol-5-yloxy)-ethylamino]-methyl}-pyridin-3-yl)-benzonitrildihydrochlorid
bb) [5-(2,4-Difluorphenyl)-pyridin-3-ylmethyl]-[2-(2-methylbenzothiazol-5-yloxy)-ethyl]-amindihydrochlorid
cc) [5-(2,4-Difluorphenyl)-pyridin-3-ylmethyl]-[2-(2-methyl-1*H-*indol-4-yloxy)-ethyl]-amindihydrochlorid
dd) [2-(2-Methyl-1*H-*indol-4-yloxy)-ethyl]-(5-p-tolyl-pyridin-3-ylmethyl)-amindihydrochlorid
ee) 7-(2-{[5-(2,4-Difluor-phenyl)-pyridin-3-ylmethyl]-amino}-ethoxy)-1*H-*indol-2-carbonsäureethylesterdihydrochlorid
ff) 7-(2-{[5-(3-Cyano-phenyl)-pyridin-3-ylmethyl]-amino}-ethoxy)-1*H-*indol-2-carbonsäureethylesterdihydrochlorid
gg) 7-(2-{[5-(3-Fluorphenyl)-pyridin-3-ylmethyl]-amino}-ethoxy)-1*H-*indol-2-carbonsäureethylesterdihydrochlorid
hh) 7-{2-[(5-p-Tolyl-pyridin-3-ylmethyl)-amino]-ethoxy}-1*H-*indol-2-carbonsäureethylesterdihydrochlorid
ii) [5-(4-Fluorphenyl)-pyridin-3-ylmethyl]-[2-(2-methyl-1*H-*indol-4-yloxy)-ethyl]-amindihydrochlorid
jj) 6-(2-{[5-(4-Fluorphenyl)-pyridin-3-ylmethyl]-amino}-ethoxy)-chromen-2-ondihydrochlorid
kk) [5-(4-Fluorphenyl)-pyridin-3-ylmethyl]-[2-(pyridin-2-yloxy)-ethyl]-amintrihydrochlorid
ll) [5-(4-Fluorphenyl)-pyridin-3-ylmethyl]-[2-(2-methylchinolin-8-yloxy)-ethyl]-amintrihydrochlorid
mm) [5-(4-Fluorphenyl)-pyridin-3-ylmethyl]-[2-(chinolin-8-yloxy)-ethyl]-amintrihydrochlorid
nn) [5-(4-Fluorphenyl)-pyridin-3-ylmethyl]-[2-(isochinolin-5-yloxy)-ethyl]-amintrihydrochlorid
oo) 5-(2-{[5-(4-Fluorphenyl)-pyridin-3-ylmethyl]-amino}-ethoxy)-1-methyl-1,3-dihydroindol-2-ondihydrochlorid
pp) [5-(4-Fluorphenyl)-pyridin-3-ylmethyl]-[2-(1*H-*indol-4-yloxy)-ethyl]-aminhydrochlorid
qq) [5-(3-Fluorphenyl)-pyridin-3-ylmethyl]-[2-(1*H-*indol-4-yloxy)-ethyl]-aminhydrochlorid
rr) [5-(2,3-Difluorphenyl)-pyridin-3-ylmethyl]-[2-(2-methyl-1*H-*indol-4-yloxy)-ethyl]-aminhydrochlorid
ss) [5-(3,5-Difluorphenyl)-pyridin-3-ylmethyl]-[2-(2-methyl-1*H-*indol-4-yloxy)-ethyl]-aminhydrochlorid
tt) [5-(2,6-Difluorphenyl)-pyridin-3-ylmethyl]-[2-(2-methyl-1*H-*indol-4-yloxy)-ethyl]-aminhydrochlorid
uu) [5-(3,4-Difluorphenyl)-pyridin-3-ylmethyl]-[2-(2-methyl-1*H-*indol-4-yloxy)-ethyl]-aminhydrochlorid
vv) [2-(2-Methyl-1*H-*indol-4-yloxy)-ethyl]-[5-(3,4,5-trifluorphenyl)-pyridin-3-ylmethyl]-aminhydrochlorid
ww) [5-(2,4-Difluoro-phenyl)-pyridin-3-ylmethyl]-[2-(1*H-*indol-4-yloxy)-ethyl]-aminhydrochlorid
xx) [2-(2-Methyl-1*H-*indol-4-yloxy)-ethyl]-(5-phenylpyridin-3-ylmethyl)-aminhydrochlorid
yy) [5-(2-Fluorphenyl)-pyridin-3-ylmethyl]-[2-(2-methyl-1*H-*indol-4-yloxy)-ethyl]-aminhydrochlorid
zz) [5-(3-Fluorphenyl)-pyridin-3-ylmethyl]-[2-(2-methyl-1H-indol-4-yloxy)-ethyl]-amin-Methansulfonat
aaa) [5-(2,5-Difluorphenyl)-pyridin-3-ylmethyl]-[2-(2-methyl-1*H-*indol-4-yloxy)-ethyl]-amin-hydrochlorid
bbb) [5-(4-Fluorphenyl)-pyridin-3-ylmethyl]-[2-(1*H-*indol-7-yloxy)-ethyl]-amin-hydrochlorid
ccc) [5-(2,4-Difluorphenyl)-pyridin-3-ylmethyl]-[2-(1*H-*indol-7-yloxy)-ethyl]-amin-hydrochlorid
ddd) [5-(3-Fluorphenyl)-pyridin-3-ylmethyl]-[2-(1*H-*indol-7-yloxy)-ethyl]-amin-hydrochlorid
eee) [5-(3,4-Difluorphenyl)-pyridin-3-ylmethyl]-[2-(1*H-*indol-4-yloxy)-ethyl]-amin-hydrochlorid
fff) [5-(3,5-Difluorphenyl)-pyridin-3-ylmethyl]-[2-(1*H-*indol-4-yloxy)-ethyl]-amin-hydrochlorid
ggg) [5-(2,5-Difluorphenyl)-pyridin-3-ylmethyl]-[2-(1*H-*indol-4-yloxy)-ethyl]-amin-hydrochlorid
hhh) [5-(3-Fluorphenyl)-pyridin-3-ylmethyl]-[2-(2-methyl-1*H-*indol-4-yloxy)-ethyl]-amin-Malonat
iii) [5-(3-Fluorphenyl)-pyridin-3-ylmethyl]-[2-(2-methyl-1*H-*indol-4-yloxy)-ethyl]-amin-Hemifumarat
jjj) [5-(3-Fluorphenyl)-pyridin-3-ylmethyl]-[2-(2-methyl-1*H-*indol-4-yloxy)-ethyl]-amin-Hemisulfat
kkk) [5-(3-Fluorphenyl)-pyridin-3-ylmethyl]-[2-(2-methyl-1*H-*indol-4-yloxy)-ethyl]-amin-Tartrat
lll) [5-(3,5-Difluorphenyl)-pyridin-3-ylmethyl]-[2-(2-methyl-1*H-*indol-4-yloxy)-ethyl]-amin-Hemifumarat
mmm) [5-(2-Fluorphenyl)-pyridin-3-ylmethyl]-[2-(1*H-*indol-4-yloxy)-ethyl]-amin-dihydrochlorid
nnn) [2-(1*H-*Indol-4-yloxy)-ethyl]-[5-(3,4,5-trifluorphenyl)-pyridin-3-ylmethyl]-amin-dihydrochlorid
ooo) [2-(1*H-*Indol-4-yloxy)-ethyl]-(5-phenyl-pyridin-3-ylmethyl)-amindihydrochlorid
sowie die korrespondierenden freien Basen sowie andere verträgliche Salze und Solvate der korrespondierenden freien Basen.

7. Verbindung der allgemeinen Formel II 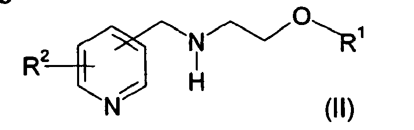 wobei R¹ und R² die Bedeutungen gemäß Anspruch 1 haben.

8. Verbindung der allgemeinen Formel III 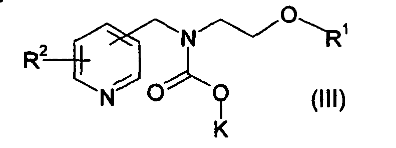 wobei R¹ und R² die Bedeutungen gemäß Anspruch 1 haben und K *tert-*Butyl, Fluoren-9-yl-methyl oder Benzyl bedeutet.

9. Verbindungen der allgemeinen Formel IV 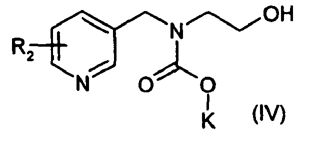 wobei R² und K die Bedeutungen gemäß Anspruch 8 haben.

10. Verbindungen der allgemeinen Formel V 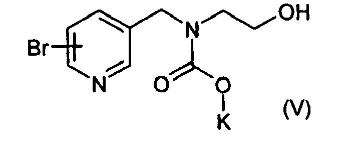 wobei K die Bedeutungen gemäß Anspruch 8 hat.

11. Verbindungen der Formel VI

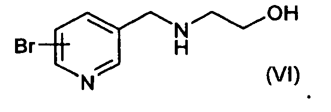

12. Verfahren zur Herstellung einer Verbindung der allgemeinen Formel I gemäß Anspruch 1, **gekennzeichnet durch** einen der folgenden Schritte:
- Synthese einer Verbindung der allgemeinen Formel VI gemäß Anspruch 11 aus Brompyridin-3-yl-methanal und H₂N-CH₂-CH₂-OH;
- Synthese einer Verbindung der allgemeinen Formel V gemäß Anspruch 10 aus L-CO-O-K und einer Verbindung der allgemeinen Formel VI gemäß Anspruch 11;
- Synthese einer Verbindung der allgemeinen Formel IV gemäß Anspruch 9 aus R²-B(OH)₂ und einer Verbindung der allgemeinen Formel V gemäß Anspruch 10;
- Synthese einer Verbindung der allgemeinen Formel III gemäß Anspruch 8 aus HO-R¹ und einer Verbindung der allgemeinen Formel IV gemäß Anspruch 9 unter den Bedingungen der Mitsunobu-Reaktion;
- Synthese einer Verbindung der allgemeinen Formel II gemäß Anspruch 7 aus einer Verbindung der allgemeinen Formel III gemäß Anspruch 8,
wobei R¹, R² und K die Bedeutungen gemäß Anspruch 8 haben und L Cl, N₃ oder *tert-*Butoxycarbonyloxy darstellt.

13. Verbindungen der allgemeinen Formel IX 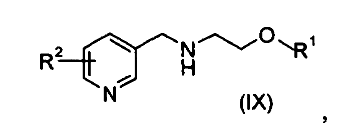 wobei R¹ und R² die in Anspruch 1 angegebenen Bedeutungen haben.

14. Verfahren zur Herstellung einer Verbindung der allgemeinen Formel I gemäß Anspruch 1, **gekennzeichnet durch** einen der folgenden Schritte:
- Synthese einer Verbindung der allgemeinen Formel XI 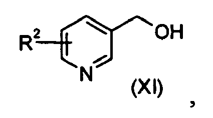 wobei R² die in Anspruch 1 angegebenen Bedeutungen hat, aus einer Verbindung der allgemeinen Formel XII 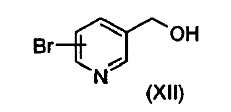 und R²-B(OH)₂, wobei R² die Bedeutungen gemäß Anspruch 1 hat;
- Synthese einer Verbindung der allgemeinen Formel X 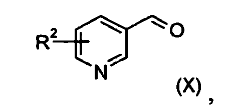 wobei R² die in Anspruch 1 angegebenen Bedeutungen hat, aus einer Verbindung der allgemeinen Formel Xl;
- Synthese einer Verbindung der allgemeinen Formel IX gemäß Anspruch 13 aus einer Verbindung der allgemeinen Formel X und R¹-O-CH₂-CH₂-NH₂, wobei R¹ die Bedeutungen gemäß Anspruch 1 besitzt;
- Synthese einer Verbindung NH₂-CH₂-CH₂-O-R¹ identisch mit einer Verbindung der allgemeinen Formel XIII 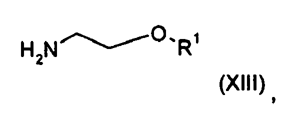 wobei R¹ die in Anspruch 1 aufgeführten Bedeutungen hat, aus einer Verbindung der allgemeinen Formel XIV 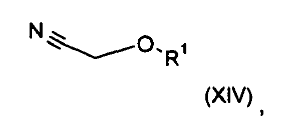 wobei R¹ die in Anspruch 1 aufgeführten Bedeutungen hat;
- Synthese einer Verbindung der allgemeinen Formel XIV aus einer Verbindung der Formel XV

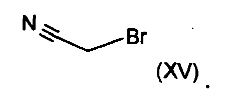

15. Verfahren zur Herstellung einer pharmazeutischen Zubereitung, **dadurch gekennzeichnet, daß** eine Verbindung nach einem der Ansprüche 1 bis 7 zusammen mit einem geeigneten Träger in eine geeignete Dosierungsform überführt wird.

16. Arzneimittel, enthaltend eine Verbindung nach einem der Ansprüche 1 bis 6.

17. Verwendung einer Verbindung nach einem der Ansprüche 1 bis 6 zur Herstellung eines Arzneimittels zur Behandlung von Krankheiten des zentralen Nervensystems, insbesondere von Geisteskrankheiten aus dem Formenkreis der Schizophrenie und zur Bekämpfung von psychotischen Angstzuständen.

## Claims

1. Compound of the general formula I 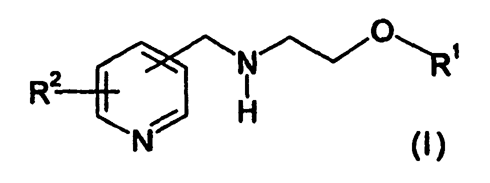 where
R¹ denotes the radical of a heterocycle having 1 to 3 ring structures, where each ring structure is saturated, unsaturated or aromatic and is optionally fused to other ring structures to form a condensed ring system, and the heterocycle has in total 1 to 4 N, O and/or S atoms in the ring structures and is optionally mono-, di- or trisubstituted by one or more of the groups -A, -OR⁴, -N(R⁴)₂, -NO₂, -CN, Hal, -COOR⁴, -CON(R⁴)_{2,} -COR⁴, =O;
R² denotes a phenyl group, which is optionally mono-, di-, tri-, tetra- or pentasubstituted by one or more of the groups Hal, A, -O-A, -NO₂ or -CN, or a thienyl group, which is optionally mono- or disubstituted by one or more of the groups Hal, A, -O-A, -NO₂, -CN or thienyl;
R⁴ denotes H or A;
A denotes C₁-C₆-alkyl, where 1 to 7 hydrogen atoms have optionally been replaced by fluorine;
Hal denotes F, Cl, Br or I;
and tolerated salts and solvates thereof.

2. Compound according to Claim 1, **characterised in that** the substituent -CH₂-NH-CH₂-CH₂-O-R¹ is in the meta position to the nitrogen of the pyridine ring.

3. Compound according to one of Claims 1 or 2, **characterised in that** R² is in the meta position to the nitrogen of the pyridine ring connected to R².

4. Compound according to one of Claims 1 to 3, **characterised by** at least one of the following features:
R¹ denotes quinolyl, isoquinolyl, which optionally have at least one chlorine substituent, or indolyl, benzothiazolyl, coumaronyl, couma- rinyl, pyridyl or carbazolyl, where at least one hydrogen of the quinolyl, isoquinolyl, indolyl or benzothiazolyl has optionally been replaced by a methyl group or ethoxycarbonyl group;
R² denotes fluorophenyl, difluorophenyl, cyanophenyl or tolyl.

5. Compound according to one of Claims 1 to 4, **characterised by** at least one of the following features:
R¹ denotes quinolin-7-yl, quinolin-8-yl, where a hydrogen in position 5 has optionally been replaced by a chlorine atom, or indol-4-yl or indol- 7-yl, where a hydrogen in position 2 of the quinolyl or indolyl has optionally been replaced by a methyl group or ethoxycarbonyl group;
R² denotes fluorophenyl, 2,4-difluorophenyl, 3-cyanophenyl or 4-methyl- phenyl.

6. Compound according to Claim 1, selected from
a) [5-(4-fluorophenyl)pyridin-3-ylmethyl]-[2-(2-methyl-1*H-*indol-4-yloxy)-ethyl]amine hydrochloride
b) [2-(5-chloroquinolin-8-yloxy)ethyl]-[5-(2,4-difluorophenyl)pyridin-3-ylmethyl]amine hydrochloride
c) [5-(2,4-difluorophenyl)pyridin-3-ylmethyl]-[2-(2-methylquinolin-8-yloxy)ethyl]amine hydrochloride
d) [5-(2,4-difluorophenyl)pyridin-3-ylmethyl]-[2-(quinolin-8-yloxy)ethyl]-amine hydrochloride
e) [2-(5-chloroquinolin-8-yloxy)ethyl]-[5-(4-fluorophenyl)pyridin-3-yl-methyl]amine hydrochloride
f) [5-(4-fluorophenyl)pyridin-3-ylmethyl]-[2-(2-methylquinolin-8-yloxy)-ethyl]amine hydrochloride
g) [5-(4-fluorophenyl)pyridin-3-ylmethyl]-[2-(quinolin-8-yloxy)ethyl]-amine hydrochloride
h) [5-(3-fluorophenyl)pyridin-3-ylmethyl]-[2-(quinolin-8-yloxy)ethyl]-amine hydrochloride
i) [5-(3-fluorophenyl)pyridin-3-ylmethyl]-[2-(2-methylquinolin-8-yloxy)-ethyl]amine hydrochloride
j) [2-(5-chloroquinolin-8-yloxy)ethyl]-[5-(3-fluorophenyl)pyridin-3-ylmethyl]amine hydrochloride
k) [5-(2,4-difluorophenyl)pyridin-3-ylmethyl]-[2-(2-methyl-1*H-*indol-4-yloxy)ethyl]amine hydrochloride
l) [5-(3-fluorophenyl)pyridin-3-ylmethyl]-[2-(2-methyl-1*H-*indol-4-yloxy)-ethyl]amine hydrochloride
m) [5-(2,4-difluorophenyl)pyridin-3-ylmethyl]-[2-(quinolin-7-yloxy)ethyl]-amine hydrochloride
n) [5-(3-fluorophenyl)pyridin-3-ylmethyl]-[2-(quinolin-7-yloxy)ethyl]-amine hydrochloride
o) [5-(4-fluorophenyl)pyridin-3-ylmethyl]-[2-(quinolin-7-yloxy)ethyl]-amine hydrochloride
p) [5-(4-fluorophenyl)pyridin-3-ylmethyl]-[2-(2-methylbenzothiazol-5-yloxy)ethyl]amine dihydrochloride
q) [5-(4-fluorophenyl)pyridin-3-ylmethyl]-[2-(quinolin-6-yloxy)ethyl]-amine trihydrochloride
r) [5-(4-fluorophenyl)pyridin-3-ylmethyl]-[2-(isoquinolin-7-yloxy)ethyl]-amine trihydrochloride
s) [2-(benzo-1,2,5-thiadiazol-5-yloxy)ethyl]-[5-(4-fluorophenyl)pyridin-3-ylmethyl]amine dihydrochloride
t) [2-(9*H-*carbazol-4-yloxy)ethyl]-[5-(4-fluorophenyl)pyridin-3-ylmethyl]-amine dihydrochloride
u) [2-(benzo-1,3-dioxol-5-yloxy)ethyl]-[5-(4-fluorophenyl)pyridin-3-ylmethyl]amine dihydrochloride
v) [2-(benzofuran-5-yloxy)ethyl]-[5-(4-fluorophenyl)pyridin-3-ylmethyl]-amine dihydrochloride
w) [2-(chroman-6-yloxy)ethyl]-[5-(4-fluorophenyl)pyridin-3-ylmethyl]-amine dihydrochloride
x) [5-(3-fluorophenyl)pyridin-3-ylmethyl]-[2-(2-methylbenzothiazol-5-yloxy)ethyl]amine dihydrochloride
y) [5-(3-fluorophenyl)pyridin-3-ylmethyl]-[2-(2-methyl-1*H-*indol-4-yloxy)-ethyl]amine dihydrochloride
z) [2-(2-methylbenzothiazol-5-yloxy)ethyl]-(5-p-tolylpyridin-3-ylmethyl)-amine dihydrochloride
aa) 3-(5-{[2-(2-methylbenzothiazol-5-yloxy)ethylamino]methyl}pyridin-3-yl)benzonitrile dihydrochloride
bb) [5-(2,4-difluorophenyl)pyridin-3-ylmethyl]-[2-(2-methylbenzothiazol-5-yloxy)ethyl]amine dihydrochloride
cc) [5-(2,4-difluorophenyl)pyridin-3-ylmethyl]-[2-(2-methyl-1*H-*indol-4-yloxy)ethyl]amine dihydrochloride
dd) [2-(2-methyl-1*H-*indol-4-yloxy)ethyl]-(5-p-tolylpyridin-3-ylmethyl)-amine dihydrochloride
ee) ethyl 7-(2-{[5-(2,4-difluorophenyl)pyridin-3-ylmethyl]amino}ethoxy)-1*H-*indole-2-carboxylate dihydrochloride
ff) ethyl 7-(2-{[5-(3-cyanophenyl)pyridin-3-ylmethyl]amino}ethoxy)-1*H-*indole-2-carboxylate dihydrochloride
gg) ethyl 7-(2-{[5-(3-fluorophenyl)pyridin-3-ylmethyl]amino}ethoxy)-1*H-*indole-2-carboxylate dihydrochloride
hh) ethyl 7-(2-[(5-p-tolylpyridin-3-ylmethyl)amino]ethoxy)-l*H-*indole-2-carboxylate dihydrochloride
ii) [5-(4-fluorophenyl)pyridin-3-ylmethyl]-[2-(2-methyl-1*H-*indol-4-yloxy)-ethyl]amine dihydrochloride
jj) 6-(2-{[5-(4-fluorophenyl)pyridin-3-ylmethyl]amino}ethoxy)chromen-2-one dihydrochloride
kk) [5-(4-fluorophenyl)pyridin-3-ylmethyl]-[2-(pyridin-2-yloxy)ethyl]amine trihydrochloride
ll) [5-(4-fluorophenyl)pyridin-3-ylmethyl]-[2-(2-methylquinolin-8-yloxy)-ethyl]amine trihydrochloride
mm) [5-(4-fluorophenyl)pyridin-3-ylmethyl]-[2-(quinolin-8-yloxy)ethyl]-amine trihydrochloride
nn) [5-(4-fluorophenyl)pyridin-3-ylmethyl]-[2-(isoquinolin-5-yloxy)ethyl]-amine trihydrochloride
oo) 5-(2-{[5-(4-fluorophenyl)pyridin-3-ylmethyl]amino}ethoxy)-1-methyl-1,3-dihydroindol-2-one dihydrochloride
pp) [5-(4-fluorophenyl)pyridin-3-ylmethyl]-[2-(1*H-*indol-4-yloxy)ethyl]-amine hydrochloride
qq) [5-(3-fluorophenyl)pyridin-3-ylmethyl]-[2-(1*H-*indol-4-yloxy)ethyl]-amine hydrochloride
rr) [5-(2,3-difluorophenyl)pyridin-3-ylmethyl]-[2-(2-methyl-1*H-*indol-4-yloxy)ethyl]amine hydrochloride
ss) [5-(3,5-difluorophenyl)pyridin-3-ylmethyl]-[2-(2-methyl-1*H-*indol-4-yloxy)ethyl]amine hydrochloride
tt) [5-(2,6-difluorophenyl)pyridin-3-ylmethyl]-[2-(2-methyl-1*H-*indol-4-yloxy)ethyl]amine hydrochloride
uu) [5-(3,4-difluorophenyl)pyridin-3-ylmethyl]-[2-(2-methyl-1*H-*indol-4-yloxy)ethyl]amine hydrochloride
vv) [2-(2-methyl-1*H-*indol-4-yloxy)ethyl]-[5-(3,4,5-trifluorophenyl)pyridin-3-ylmethyl]amine hydrochloride
ww) [5-(2,4-difluorophenyl)pyridin-3-ylmethyl]-[2-(1*H-*indol-4-yloxy)ethyl]-amine hydrochloride
xx) [2-(2-methyl-1*H-*indol-4-yloxy)ethyl]-(5-phenylpyridin-3-ylmethyl)-amine hydrochloride
yy) [5-(2-fluorophenyl)pyridin-3-ylmethyl]-[2-(2-methyl-1*H-*indol-4-yloxy)-ethyl]amine hydrochloride
zz) [5-(3-fluorophenyl)pyridin-3-ylmethyl]-[2-(2-methyl-1*H-*indol-4-yloxy)-ethyl]amine methanesulfonate
aaa) [5-(2,5-difluorophenyl)pyridin-3-ylmethyl]-[2-(2-methyl-1*H-*indol-4-yloxy)ethyl]amine hydrochloride
bbb) [5-(4-fluorophenyl)pyridin-3-ylmethyl]-[2-(1*H-*indol-7-yloxy)ethyl]-amine hydrochloride
ccc) [5-(2,4-difluorophenyl)pyridin-3-ylmethyl]-[2-(1*H-*indol-7-yloxy)ethyl]-amine hydrochloride
ddd) [5-(3-fluorophenyl)pyridin-3-ylmethyl]-[2-(1*H-*indol-7-yloxy)ethyl]-amine hydrochloride
eee) [5-(3,4-difluorophenyl)pyridin-3-ylmethyl]-[2-(1*H-*indol-4-yloxy)ethyl]-amine hydrochloride
fff) [5-(3,5-difluorophenyl)pyridin-3-ylmethyl]-[2-(1*H-*indol-4-yloxy)ethyl]-amine hydrochloride
ggg) [5-(2,5-difluorophenyl)pyridin-3-ylmethyl]-[2-(1*H-*indol-4-yloxy)ethyl]-amine hydrochloride
hhh) [5-(3-fluorophenyl)pyridin-3-ylmethyl]-[2-(2-methyl-1*H-*indol-4-yloxy)-ethyl]amine malonate
iii) [5-(3-fluorophenyl)pyridin-3-ylmethyl]-[2-(2-methyl-1*H-*indol-4-yloxy)-ethyl]amine hemifumarate
jjj) [5-(3-fluorophenyl)pyridin-3-ylmethyl]-[2-(2-methyl-1*H-*indol-4-yloxy)-ethyl]amine hemisulfate
kkk) [5-(3-fluorophenyl)pyridin-3-ylmethyl]-[2-(2-methyl-1*H-*indol-4-yloxy)-ethyl]amine tartrate
lll) [5-(3,5-difluorophenyl)pyridin-3-ylmethyl]-[2-(2-methyl-1*H-*indol-4-yloxy)ethyl]amine hemifumarate
mmm) [5-(2-fluorophenyl)pyridin-3-ylmethyl]-[2-(1*H-*indol-4-yloxy)ethyl]-amine dihydrochloride
nnn) [2-(1*H-*indol-4-yloxy)ethyl]-[5-(3,4,5-trifluorophenyl)pyridin-3-yl-methyl]amine dihydrochloride
ooo) [2-(1*H-*indol-4-yloxy)ethyl]-(5-phenylpyridin-3-ylmethyl)amine dihydrochloride
and the corresponding free bases and other tolerated salts and solvates of the corresponding free bases.

7. Compound of the general formula II 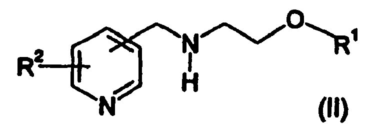 where R¹ and R² have the meanings according to Claim 1.

8. Compound of the general formula III 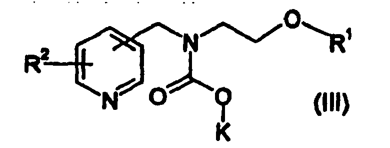 where R¹ and R² have the meanings according to Claim 1, and K denotes *tert-*butyl, fluoren-9-ylmethyl or benzyl.

9. Compounds of the general formula IV 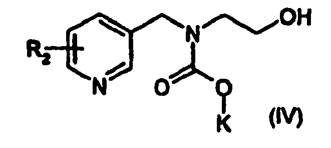 where R² and K have the meanings according to Claim 8.

10. Compounds of the general formula V 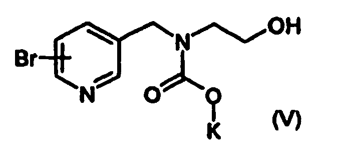 where K has the meanings according to Claim 8.

11. Compounds of the formula VI

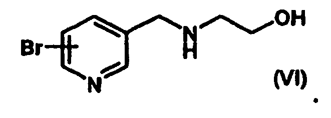

12. Process for the preparation of a compound of the general formula I according to Claim 1, **characterised by** one of the following steps:
- synthesis of a compound of the general formula VI according to Claim 11 from bromopyridin-3-ylmethanal and H₂N-CH₂-CH₂-OH;
- synthesis of a compound of the general formula V according to Claim 10 from L-CO-O-K and a compound of the general formula VI according to Claim 11;
- synthesis of a compound of the general formula IV according to Claim 9 from R²-B(OH)₂ and a compound of the general formula V according to Claim 10;
- synthesis of a compound of the general formula III according to Claim 8 from HO-R¹ and a compound of the general formula IV according to Claim 9 under the conditions of the Mitsunobu reaction;
- synthesis of a compound of the general formula II according to Claim 7 from a compound of the general formula III according to Claim 8,
where R¹, R² and K have the meanings according to Claim 8, and L represents Cl, N₃ or *tert-*butoxycarbonyloxy.

13. Compounds of the general formula IX 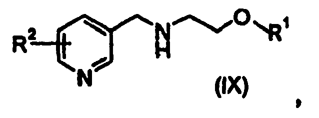 where R¹ and R² have the meanings indicated in Claim 1.

14. Process for the preparation of a compound of the general formula I according to Claim 1, **characterised by** one of the following steps:
- synthesis of a compound of the general formula XI 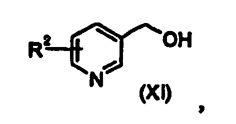 where R² has the meanings indicated in Claim 1, from a compound of the general formula XII 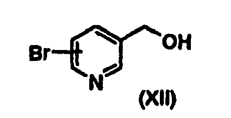 and R²-B(OH)_{2,} where R² has the meanings according to Claim 1;
- synthesis of a compound of the general formula X 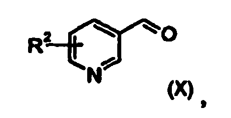 where R² has the meanings indicated in Claim 1, from a compound of the general formula XI;
- synthesis of a compound of the general formula IX according to Claim 13 from a compound of the general formula X and R¹-O-CH₂-CH₂-NH₂, where R¹ has the meanings according to Claim 1;
- synthesis of a compound NH₂-CH₂-CH₂-O-R¹, identical to a compound of the general formula XIII 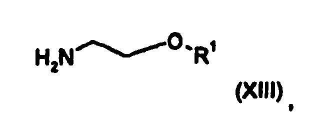 where R¹ has the meanings given in Claim 1, from a compound of the general formula XIV 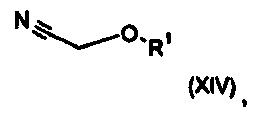 where R¹ has the meanings given in Claim 1;
- synthesis of a compound of the general formula XIV from a compound of the formula XV

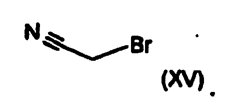

15. Process for the preparation of a pharmaceutical composition, **characterised in that** a compound according to one of Claims 1 to 6 is converted into a suitable dosage form together with a suitable excipient.

16. Medicament comprising a compound according to one of Claims 1 to 6.

17. Use of a compound according to one of Claims 1 to 6 for the preparation of a medicament for the treatment of diseases of the central nervous system, in particular mental illnesses of the schizophrenia type, and for combating psychotic anxiety states.

## Revendications

1. Composé de formule générale I 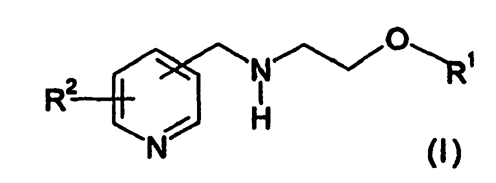 dans laquelle
R¹ désigne le radical d'un hétérocycle ayant 1 à 3 structures de cycle, où chaque structure de cycle est saturée, insaturée ou aromatique et est éventuellement condensée sur d'autres structures de cycle afin de former un noyau condensé, et l'hétérocycle possède un total de 1 à 4 atomes de N, O et/ou S dans les structures de cycle et est éventuel- lement mono-, di- ou trisubstitué par un ou plusieurs parmi les grou- pements -A, -OR⁴, -N(R⁴)₂, -NO₂, -CN, Hal, -COOR⁴, -CON(R⁴)₂, -COR⁴, =O;
R² désigne un groupement phényle, qui est éventuellement mono-, di-, tri-, tétra- ou pentasubstitué par un ou plusieurs parmi les groupe- ments Hal, A, -O-A, -NO₂ ou -CN, ou un groupement thiényle, qui est éventuellement mono- ou disubstitué par un ou plusieurs parmi les groupements Hal, A, -O-A, -NO₂, -CN ou thiényle ;
R⁴ désigne H ou A;
A désigne C₁-C₆-alkyle, où 1 à 7 atomes d'hydrogène sont éventuelle- ment remplacés par du fluor ;
Hal désigne F, Cl, Br ou I;
et les sels et solvats tolérés de celui-ci.

2. Composé selon la revendication 1, **caractérisé en ce que** le substituant -CH₂-NH-CH₂-CH₂-O-R¹ est en position méta par rapport à l'azote du cycle pyridine.

3. Composé selon l'une des revendications 1 ou 2, **caractérisé en ce que** R² est en position méta par rapport à l'azote du cycle pyridine relié à R².

4. Composé selon l'une des revendications 1 à 3, **caractérisé par** au moins l'une des caractéristiques suivantes :
R¹ désigne quinolyle, isoquinolyle, qui possèdent éventuellement au moins un substituant chlore, ou indolyle, benzothiazolyle, coumaron- yle, coumarinyle, pyridyle ou carbazolyle, où au moins un hydrogène du groupement quinolyle, isoquinolyle, indolyle ou benzothiazolyle a éventuellement été remplacé par un groupement méthyle ou un groupement éthoxycarbonyle ;
R² désigne fluorophényle, difluorophényle, cyanophényle ou tolyle.

5. Composé selon l'une des revendications 1 à 4, **caractérisé par** au moins l'une des caractéristiques suivantes :
R¹ désigne quinoléin-7-yle, quinoléin-8-yle, où un hydrogène en position 5 a éventuellement été remplacé par un atome de chlore, ou indol-4- yle ou indol-7-yle, où un hydrogène en position 2 du groupement qui- nolyle ou indolyle a éventuellement été remplacé par un groupement méthyle ou un groupement éthoxycarbonyle ;
R² désigne fluorophényle, 2,4-difluorophényle, 3-cyanophényle ou 4- méthylphényle.

6. Composé selon la revendication 1, choisi parmi
a) le chlorhydrate de [5-(4-fluorophényl)pyridin-3-ylméthyl]-[2-(2-méthyl-1*H-*indol-4-yloxy)éthyl]amine,
b) le chlorhydrate de [2-(5-chloroquinoléin-8-yloxy)éthyl]-[5-(2,4-difluorophényl)pyridin-3-ylméthyl]amine,
c) le chlorhydrate de [5-(2,4-difluorophényl)pyridin-3-ylméthyl]-[2-(2-méthylquinoléin-8-yloxy)éthyl]amine,
d) le chlorhydrate de [5-(2,4-difluorophényl)pyridin-3-ylméthyl]-[2-(quinoléin-8-yloxy)éthyl]amine,
e) le chlorhydrate de [2-(5-chloroquinoléin-8-yloxy)éthyl]-[5-(4-fluorophényl)pyridin-3-ylméthyl]amine,
f) le chlorhydrate de [5-(4-fluorophényl)pyridin-3-ylméthyl]-[2-(2-méthylquinoléin-8-yloxy)éthyl]amine,
g) le chlorhydrate de [5-(4-fluorophényl)pyridin-3-ylméthyl]-[2-(quinoléin-8-yloxy)éthyl]amine,
h) le chlorhydrate de [5-(3-fluorophényl)pyridin-3-ylméthyl]-[2-(quinoléin-8-yloxy)éthyl]amine,
i) le chlorhydrate de [5-(3-fluorophényl)pyridin-3-ylméthyl]-[2-(2-méthylquinoléin-8-yloxy)éthyl]amine,
j) le chlorhydrate de [2-(5-chloroquinoléin-8-yloxy)éthyl]-[5-(3-fluorophényl)pyridin-3-ylméthyl]amine,
k) le chlorhydrate de [5-(2,4-difluorophényl)pyridin-3-ylméthyl]-[2-(2-méthyl-1*H-*indol-4-yloxy)éthyl]amine,
l) le chlorhydrate de [5-(3-fluorophényl)pyridin-3-ylméthyl]-[2-(2-méthyl-1*H-*indol-4-yloxy)éthyl]amine,
m) le chlorhydrate de [5-(2,4-difluorophényl)pyridin-3-ylméthyl]-[2-(quinoléin-7-yloxy)éthyl]amine,
n) le chlorhydrate de [5-(3-fluorophényl)pyridin-3-ylméthyl]-[2-(quinoléin-7-yloxy)éthyl]amine,
o) le chlorhydrate de [5-(4-fluorophényl)pyridin-3-ylméthyl]-[2-(quinoléin-7-yloxy)éthyl]amine,
p) le dichlorhydrate de [5-(4-fluorophényl)pyridin-3-ylméthyl]-[2-(2-méthylbenzothiazol-5-yloxy)éthyl]amine,
q) le trichlorhydrate de [5-(4-fluorophényl)pyridin-3-ylméthyl]-[2-(quinoléin-6-yloxy)éthyl]amine,
r) le trichlorhydrate de [5-(4-fluorophényl)pyridin-3-ylméthyl]-[2-(isoquinoléin-7-yloxy)éthyl]amine,
s) le dichlorhydrate de [2-(benzo-1,2,5-thiadiazol-5-yloxy)éthyl]-[5-(4-fluorophényl)pyridin-3-ylméthyl]amine,
t) le dichlorhydrate de [2-(9*H-*carbazol-4-yloxy)éthyl]-[5-(4-fluorophényl)pyridin-3-ylméthyl]amine,
u) le dichlorhydrate de [2-(benzo-1,3-dioxol-5-yloxy)éthyl]-[5-(4-fluorophényl)pyridin-3-ylméthyl]amine,
v) le dichlorhydrate de [2-(benzofuran-5-yloxy)éthyl]-[5-(4-fluorophényl)pyridin-3-ylméthyl]amine,
w) le dichlorhydrate de [2-(chroman-6-yloxy)éthyl]-[5-(4-fluorophényl)-pyridin-3-ylméthyl]amine,
x) le dichlorhydrate de [5-(3-fluorophényl)pyridin-3-ylméthyl]-[2-(2-méthylbenzothiazol-5-yloxy)éthyl]amine,
y) le dichlorhydrate de [5-(3-fluorophényl)pyridin-3-ylméthyl]-[2-(2-méthyl-1*H-*indol-4-yloxy)éthyl]amine,
z) le dichlorhydrate de [2-(2-méthylbenzothiazol-5-yloxy)éthyl]-(5-p-tolylpyridin-3-ylméthyl)amine,
aa) le dichlorhydrate de 3-(5-{[2-(2-méthylbenzothiazol-5-yloxy)éthylamino]méthyl}pyridin-3-yl)benzonitrile,
bb) le dichlorhydrate de [5-(2,4-difluorophényl)pyridin-3-ylméthyl]-[2-(2-méthylbenzothiazol-5-yloxy)éthyl]amine,
cc) le dichlorhydrate de [5-(2,4-difluorophényl)pyridin-3-ylméthyl]-[2-(2-méthyl-1*H-*indol-4-yloxy)éthyl]amine,
dd) le dichlorhydrate de [2-(2-méthyl-1*H-*indol-4-yloxy)éthyl]-(5-p-tolylpyridin-3-ylméthyl)amine,
ee) le 7-(2-{[5-(2,4-difluorophényl)pyridin-3-ylméthyl]amino}éthoxy)-1*H-*indole-2-carboxylate d'éthyle, dichlorhydrate,
ff) le 7-(2-{[5-(3-cyanophényl)pyridin-3-ylméthyl]amino}éthoxy)-1*H-*indole-2-carboxylate d'éthyle, dichlorhydrate,
gg) le 7-(2-{[5-(3-fluorophényl)pyridin-3-ylméthyl]amino}éthoxy)-1*H-*indole-2-carboxylate d'éthyle, dichlorhydrate,
hh) le 7-{2-[(5-p-tolylpyridin-3-ylméthyl)amino]éthoxy}-1*H-*indole-2-carboxylate d'éthyle, dichlorhydrate,
ii) le dichlorhydrate de [5-(4-fluorophényl)pyridin-3-ylméthyl]-[2-(2-méthyl-1*H-*indol-4-yloxy)éthyl]amine,
jj) le dichlorhydrate de 6-(2-{[5-(4-fluorophényl)pyridin-3-ylméthyl]-amino}éthoxy)chromén-2-one,
kk) le trichlorhydrate de [5-(4-fluorophényl)pyridin-3-ylméthyl]-[2-(pyridin-2-yloxy)éthyl]amine,
ll) le trichlorhydrate de [5-(4-fluorophényl)pyridin-3-ylméthyl]-[2-(2-méthylquinoléin-8-yloxy)éthyl]amine,
mm) le trichlorhydrate de [5-(4-fluorophényl)pyridin-3-ylméthyl]-[2-(quinoléin-8-yloxy)éthyl]amine,
nn) le trichlorhydrate de [5-(4-fluorophényl)pyridin-3-ylméthyl]-[2-(isoquinoléin-5-yloxy)éthyl]amine,
oo) le dichlorhydrate de 5-(2-{[5-(4-fluorophényl)pyridin-3-ylméthyl]-amino}éthoxy)-1-méthyl-1,3-dihydroindol-2-one,
pp) le chlorhydrate de [5-(4-fluorophényl)pyridin-3-ylméthyl]-[2-(1*H-*indol-4-yloxy)éthyl]amine,
qq) le chlorhydrate de [5-(3-fluorophényl)pyridin-3-ylméthyl]-[2-(1*H-*indol-4-yloxy)éthyl]amine,
rr) le chlorhydrate de [5-(2,3-difluorophényl)pyridin-3-ylméthyl]-[2-(2-méthyl-1*H-*indol-4-yloxy)éthyl]amine,
ss) le chlorhydrate de [5-(3,5-difluorophényl)pyridin-3-ylméthyl]-[2-(2-méthyl-1*H-*indol-4-yloxy)éthyl]amine,
tt) le chlorhydrate de [5-(2,6-difluorophényl)pyridin-3-ylméthyl]-[2-(2-méthyl-1*H-*indol-4-yloxy)éthyl]amine,
uu) le chlorhydrate de [5-(3,4-difluorophényl)pyridin-3-ylméthyl]-[2-(2-méthyl-1*H-*indol-4-yloxy)éthyl]amine,
vv) le chlorhydrate de [2-(2-méthyl-1*H-*indol-4-yloxy)éthyl]-[5-(3,4,5-trifluorophényl)pyridin-3-ylméthyl]amine,
ww) le chlorhydrate de [5-(2,4-difluorophényl)pyridin-3-ylméthyl]-[2-(1*H-*indol-4-yloxy)éthyl]amine,
xx) le chlorhydrate de [2-(2-méthyl-1*H-*indol-4-yloxy)éthyl]-(5-phénylpyridin-3-ylméthyl)amine,
yy) le chlorhydrate de [5-(2-fluorophényl)pyridin-3-ylméthyl]-[2-(2-méthyl-1*H-*indol-4-yloxy)éthyl]amine,
zz) le méthanesulfonate de [5-(3-fluorophényl)pyridin-3-ylméthyl]-[2-(2-méthyl-1*H-*indol-4-yloxy)éthyl]amine
aaa) le chlorhydrate de [5-(2,5-difluorophényl)pyridin-3-ylméthyl]-[2-(2-méthyl-1*H-*indol-4-yloxy)éthyl]amine,
bbb) le chlorhydrate de [5-(4-fluorophényl)pyridin-3-ylméthyl]-[2-(1*H-*indol-7-yloxy)éthyl]amine,
ccc) le chlorhydrate de [5-(2,4-difluorophényl)pyridin-3-ylméthyl]-[2-(1*H-*indol-7-yloxy)éthyl]amine,
ddd) le chlorhydrate de [5-(3-fluorophényl)pyridin-3-ylméthyl]-[2-(1*H-*indol-7-yloxy)éthylamine,
eee) le chlorhydrate de [5-(3,4-difluorophényl)pyridin-3-ylméthyl]-[2-(1*H-*indol-4-yloxy)éthyl]amine,
fff) le chlorhydrate de [5-(3,5-difluorophényl)pyridin-3-ylméthyl]-[2-(1*H-*indol-4-yloxy)éthyl]amine,
ggg) le chlorhydrate de [5-(2,5-difluorophényl)pyridin-3-ylméthyl]-[2-(1*H-*indol-4-yloxy)éthyl]amine,
hhh) le malonate de [5-(3-fluorophényl)pyridin-3-ylméthyl]-[2-(2-méthyl-1*H-*indol-4-yloxy)éthylamine,
iii) l'hémi-fumarate de [5-(3-fluorophényl)pyridin-3-ylméthyl]-[2-(2-méthyl-1*H-*indol-4-yloxy)éthyl]amine,
jjj) l'hémi-sulfate de [5-(3-fluorophényl)pyridin-3-ylméthyl]-[2-(2-méthyl-1*H-*indol-4-yloxy)éthyl]amine,
kkk) le tartrate de [5-(3-fluorophényl)pyridin-3-ylméthyl]-[2-(2-méthyl-1*H-*indol-4-yloxy)éthyl]amine,
lll) l'hémi-fumarate de [5-(3,5-difluorophényl)pyridin-3-ylméthyl]-[2-(2-méthyl-1*H-*indol-4-yloxy)éthyl]amine,
mmm) le dichlorhydrate de [5-(2-fluorophényl)pyridin-3-ylméthyl]-[2-(1*H-*indol-4-yloxy)éthyl] amine,
nnn) le dichlorhydrate de [2-(1*H-*indol-4-yloxy)éthyl]-[5-(3,4,5-trifluorophényl)pyridin-3-ylméthyl]amine,
ooo) le dichlorhydrate de [2-(1*H-*indol-4-yloxy)éthyl]-(5-phénylpyridin-3-ylméthyl)amine,
et les bases libres correspondantes et autres sels et solvats tolérés des bases libres correspondantes.

7. Composé de formule générale II 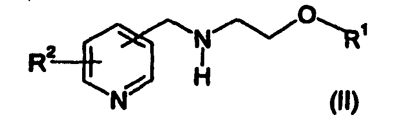 dans laquelle R¹ et R² revêtent les significations selon la revendication 1.

8. Composé de formule générale III 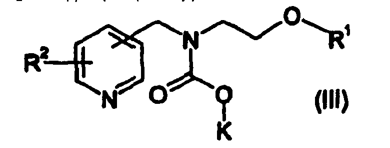 dans laquelle R¹ et R² revêtent les significations selon la revendication 1, et K désigne *tertio*-butyle, fluorén-9-ylméthyle ou benzyle.

9. Composés de formule générale IV 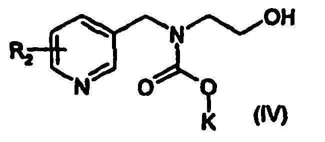 dans laquelle R² et K revêtent les significations selon la revendication 8.

10. Composés de formule générale V 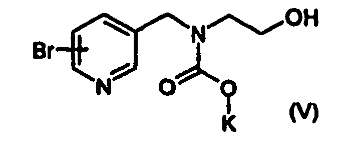 dans laquelle K revêt les significations selon la revendication 8.

11. Composés de formule VI

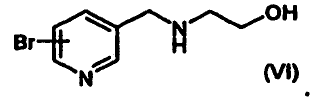

12. Procédé de préparation d'un composé de formule générale 1 selon la revendication 1, **caractérisé par** l'une des étapes suivantes :
- la synthèse d'un composé de formule générale VI selon la revendication 11 à partir de bromopyridin-3-ylméthanal et de H₂N-CH₂-CH₂-OH;
- la synthèse d'un composé de formule générale V selon la revendication 10 à partir de L-CO-O-K et d'un composé de formule générale VI selon la revendication 11;
- la synthèse d'un composé de formule générale IV selon la revendication 9 à partir de R²-B(OH)₂ et d'un composé de formule générale V selon la revendication 10;
- la synthèse d'un composé de formule générale III selon la revendication 8 à partir de HO-R¹ et d'un composé de formule générale IV selon la revendication 9 dans les conditions d'une réaction de Mitsunobu;
- la synthèse d'un composé de formule générale II selon la revendication 7 à partir d'un composé de formule générale III selon la revendication 8,
où R¹, R² et K revêtent les significations selon la revendication 8, et L représente Cl, N₃ ou *tertio*-butoxycarbonyloxy.

13. Composés de formule générale IX 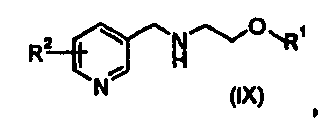 dans laquelle R¹ et R² revêtent les significations indiquées selon la revendication 1.

14. Procédé de préparation d'un composé de formule générale I selon la revendication 1, **caractérisé par** l'une des étapes suivantes :
- la synthèse d'un composé de formule générale XI 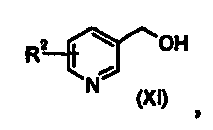 dans laquelle R² revêt les significations indiquées selon la revendication 1, à partir d'un composé de formule générale XII 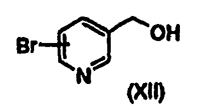 et de R²-B(OH)₂, où R² revêt les significations selon la revendication 1;
- la synthèse d'un composé de formule générale X 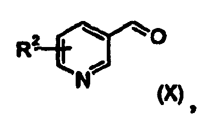 dans laquelle R² revêt les significations indiquées selon la revendication 1, à partir d'un composé de formule générale XI;
- la synthèse d'un composé de formule générale IX selon la revendication 13 à partir d'un composé de formule générale X et de R¹-O-CH₂-CH₂-NH₂, où R¹ revêt les significations selon la revendication 1;
- la synthèse d'un composé NH₂-CH₂-CH₂-O-R¹, identique à un composé de formule générale XIII 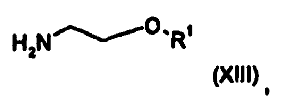 dans laquelle R¹ revêt les significations indiquées selon la revendication 1, à partir d'un composé de formule générale XIV 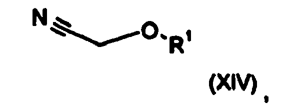 dans laquelle R¹ revêt les significations indiquées selon la revendication 1;
- la synthèse d'un composé de formule générale XIV à partir d'un composé de formule XV

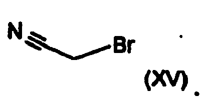

15. Procédé de préparation d'une composition pharmaceutique, **caractérisé en ce qu'**un composé selon l'une des revendications 1 à 6 est converti en une forme de dosage convenable, conjointement avec un excipient convenable.

16. Médicament comprenant un composé selon l'une des revendications 1 à 6.

17. Utilisation d'un composé selon l'une des revendications 1 à 6, pour la préparation d'un médicament destiné au traitement de maladies du système nerveux central, en particulier des maladies mentales de type schizophrénie, et pour lutter contre les états d'anxiété psychotique.
